(19) Europäisches Patentamt
European Patent Office
Office européen des brevets

(11) **EP 3 400 946 B1**

(12) **EUROPEAN PATENT SPECIFICATION**

(45) Date of publication and mention of the grant of the patent:
**07.04.2021 Bulletin 2021/14**

(21) Application number: **17844595.3**

(22) Date of filing: **07.04.2017**

(51) Int Cl.:
***A61K 31/7072*** *(2006.01)* ***A61K 31/4709*** *(2006.01)*
***A61K 31/403*** *(2006.01)* ***A61P 31/14*** *(2006.01)*
***A61P 31/12*** *(2006.01)* ***A61K 9/08*** *(2006.01)*
***A61K 9/20*** *(2006.01)* ***A61K 9/48*** *(2006.01)*

(86) International application number:
**PCT/RU2017/000211**

(87) International publication number:
**WO 2018/160090 (07.09.2018 Gazette 2018/36)**

(54) **ANTIVIRAL COMPOSITION AND METHOD FOR USING SAME**

ANTIVIRALE ZUSAMMENSETZUNG UND VERFAHREN ZUR ANWENDUNG DAVON

COMPOSITION ANTIVIRALE ET PROCÉDÉ DE SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2017 RU 2017106610**

(43) Date of publication of application:
**14.11.2018 Bulletin 2018/46**

(73) Proprietors:
- **Alla Chem, LLC**
  **Hallandale Beach, FL 33009 (US)**
- **Ivachtchenko, Alexandre Vasilievich**
  **Hallandale Beach, FL 33009 (US)**
- **Ivashchenko, Andrey Alexandrovich**
  **Moscow 127576 (RU)**
- **Ivachtchenko, Alena Alexandrovna**
  **Hallandale, Florida 33009 (US)**
- **Savchuk, Nikolay Filippovich**
  **Rancho Santa Fe, CA 92067 (US)**

(72) Inventors:
- **IVACHTCHENKO, Alexandre Vasilievich**
  **Hallandale Beach, Florida 33009 (US)**
- **MITKIN, Oleg Dmitrievich**
  **Khimki 141400 (RU)**

(74) Representative: **Zellentin & Partner mbB**
**Patentanwälte**
**Rubensstrasse 30**
**67061 Ludwigshafen (DE)**

(56) References cited:
**WO-A1-2016/141092** **EA-A1- 200 400 986**
**RU-A- 2010 123 928** **RU-C2- 2 478 104**
**US-A1- 2014 066 398**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 400 946 B1

## Description

### Field of the invention

Hepatitis C caused by hepatitis C virus (HCV) is one of the world's most widely spread liver diseases. According to the annual reports of the World Health Organization (WHO), more than 130-150 mln people are infected with HCV and more than 700 thousand individuals die from HCV [WHO. Hepatitis C. WHO fact sheet № 164. Updated July 2016, http://www.who.int/mediacentre /factsheets/ fs164/en/]. HCV demonstrates a high genetic diversity and is characterized by regional variations of (gT) HCV genotypes. Genotype 1 (gT1) is the most common in the world (83.4 mln people, or 46.2% of all HCV-infected; about one third of them are in East Asia). Genotype 3 (gT3) is the second most common genotype. Globally, 54.3 mln people (30.1%) are infected with gT3. Genotypes 2, 4, and 6 account for 22.8% of all HCV-infected people, while genotype 5 (gT5) accounts for <1%. While genotypes 1 and 3 prevail in the majority of countries regardless of their economic status, the greatest occurrence of genotypes 4 and 5 are in low-income states [Messina, J. P. at al. Global Distribution and Prevalence of Hepatitis C Virus Genotypes. Hepatology 2015, 61(1), 77-87.].

### Background of the invention

Considerable progress in the therapy of hepatitis C achieved in recent years is primarily associated with the discovery of Sofosbuvir (also, Sovaldi, PSI-7977, GS-7977), HCV NS5B nucleoside inhibitor [Sofia, M. J. et al. Discovery of a β-D-20-Deoxy-20-rfluoro-2 0-β-C-methyluridine Nucleotide Prodrug (PSI-7977) for the Treatment of Hepatitis C Virus. J. Med. Chem. 2010, 53, 7202-7218. Sofia, M. J. et al. Nucleoside phosphoramidate prodrugs. Patent US 7964580, 2011. Sofia, M. J. Nucleoside phosphoramidate prodrugs. Patent US 8334270, 2012.] and Daclatasvir (BMS-790052) a NS5A HCV inhibitor [Belema, M. et al. Discovery and Development of Hepatitis C Virus NS5A Replication Complex Inhibitors. J. Med. Chem. 2014, 57, 1643-1672. Bachand, C. et al. Hepatitis C virus inhibitors. Patent WO 2008/021927, 2008. Bachand, C. et al. Hepatitis c virus inhibitors. Patent WO 2008/021928, 2008. Bachand, C. et al. Hepatitis C virus inhibitors. Patent WO2008/021936, 2008.
http://www.ema.europa.eu/docs/en_GB/document_library/EPAR_Public_assessm ent report/human/003768/WC500172849.pdf.].

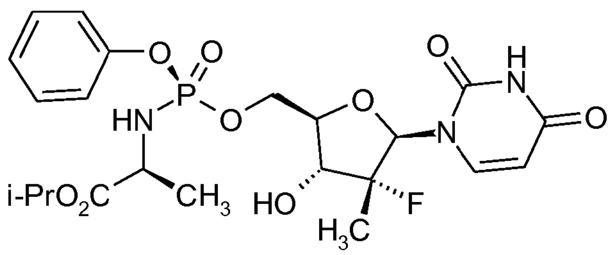

**Sovaldi (GS-7977, PSI-7977, Sofosbuvir)**

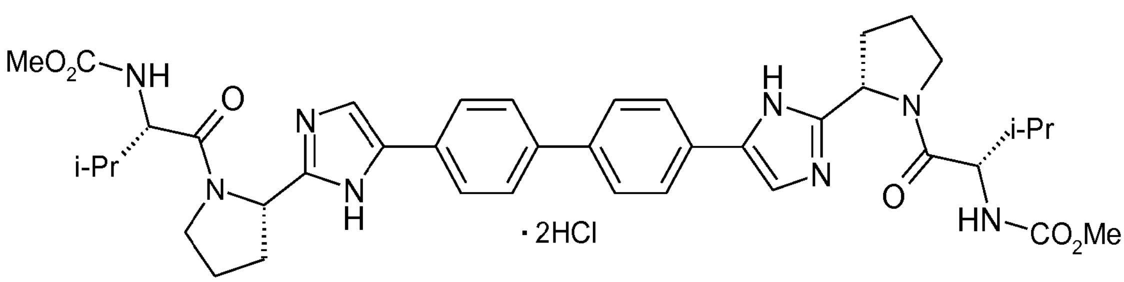

**Declatasvir (BMS-790052)**

Later, other NS5A inhibitors were obtained including Ledipasvir (GS-5885) [Link, J. O. et al. Discovery of ledipasvir (GS-5885): a potent, once-daily oral NS5A inhibitor for the treatment of hepatitis C virus infection. J. Med. Chem. 2014, 57, 2033-2046. Guo, H. et al. Antiviral compounds. Patent WO 2010/132601, 2010. http://www.ema.europa.eu/docs/en_
GB/document_library/EPAR_Public_assessment_

report/human/003850/WC500177996.pdf. http://www.hcvdruginfo.ca/downloads/HCV_ledipasvir.pdf],

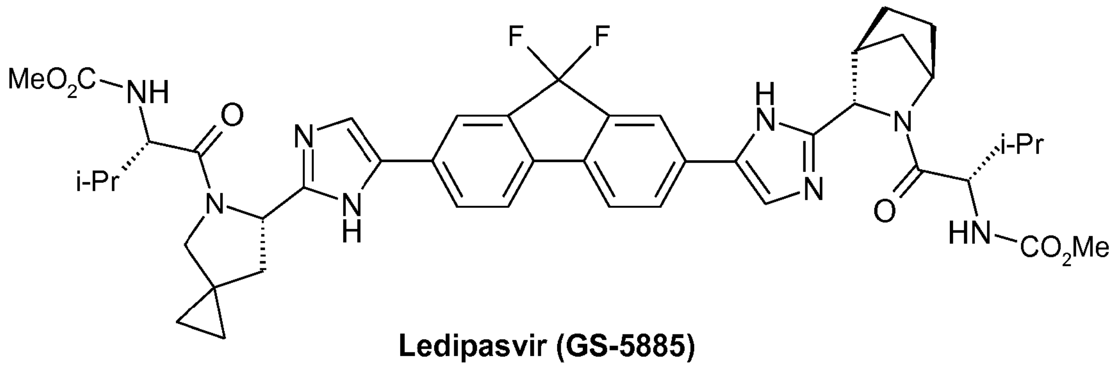

**Ledipasvir (GS-5885)**

Ombitasvir (ABT-267) [Gardelli, C. et al. Phosphoramidate Prodrugs of 20-C-Methylcytidine for Therapy of Hepatitis C Virus Infection. J. Med. Chem. 2014, 57, 2047-2057. Patent WO 2010/144646, 2010.],

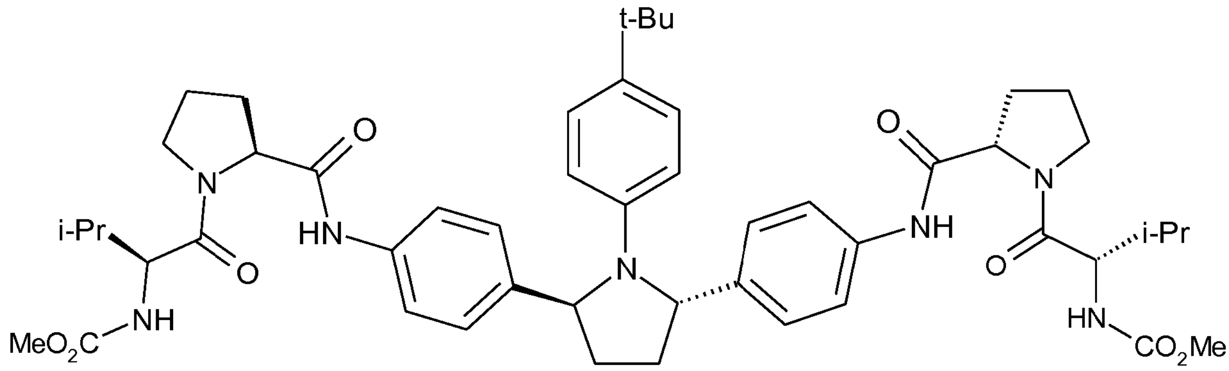

**Ombitasvir (ABT-267)**

Elbasvir (MK-8742) [Coburn, C. A. et al. ChemMedChem. 2013, 8, 1930-1940.

Patent WO 2012/040923, 2012. Patent WO 2012/041014, 2012],

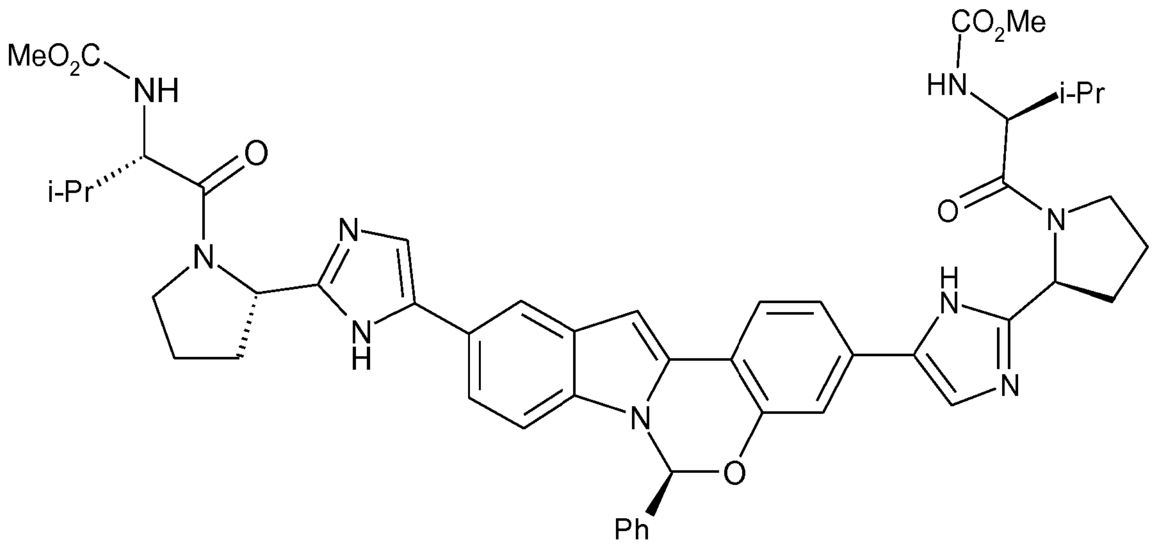

**Elbasvir (MK-8742)**

Velpatasvir (VEL, GS-5816) [Patent WO 2015/110048, 2015. http://www.accessdata.fda.gov/ drugsatfdadocs/ nda/2016/208341O rig1s000PharmR.pdf]. http://www.gilead.com/~/media/files/pdfs/ medicines/liver-disease/epclusa/epclusa_pi.pdf],

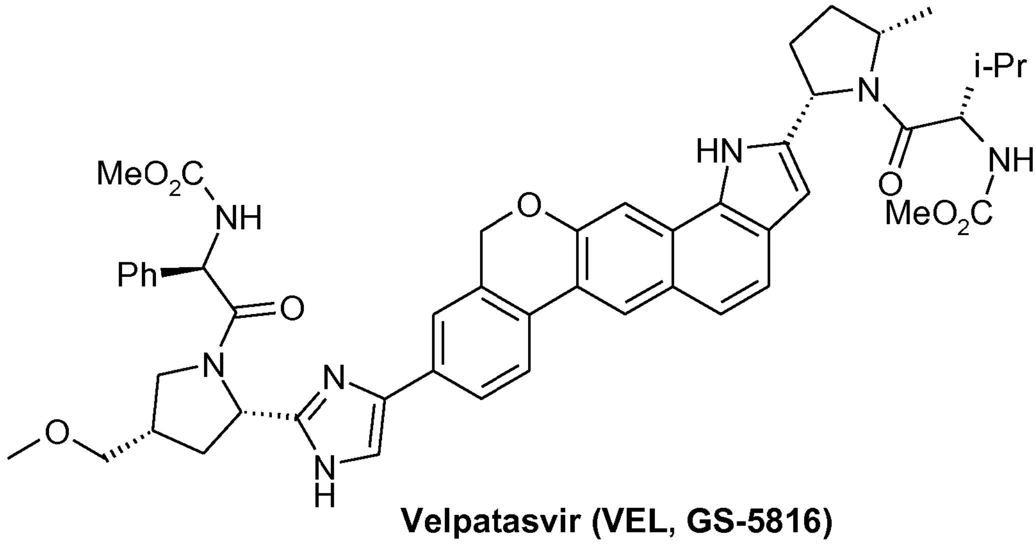

**Velpatasvir (VEL, GS-5816)**

Hepavivir (AV-4025) [Ivachtchenko, A. V. et al. Discovery of Novel Highly Potent Hepatitis C Virus NS5A Inhibitor (AV4025). J. Med. Chem. 2014, 57, 7716-7730. Patent WO 2012/074437, 2012. Patent US 9428491, 2016.], AV-4056 and AV-4058 [US 9428491.], AV-4067 and AV-4084 [Pat. Appl. US 14/845,333.],

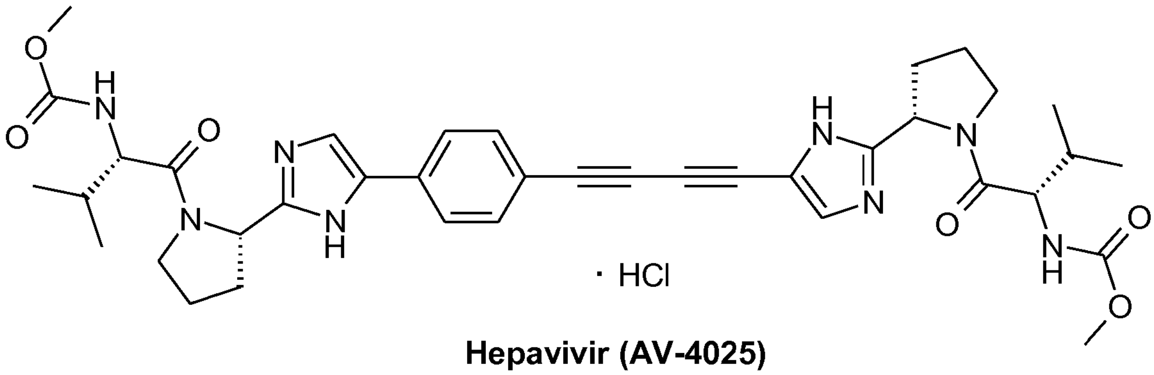

**Hepavivir (AV-4025)**

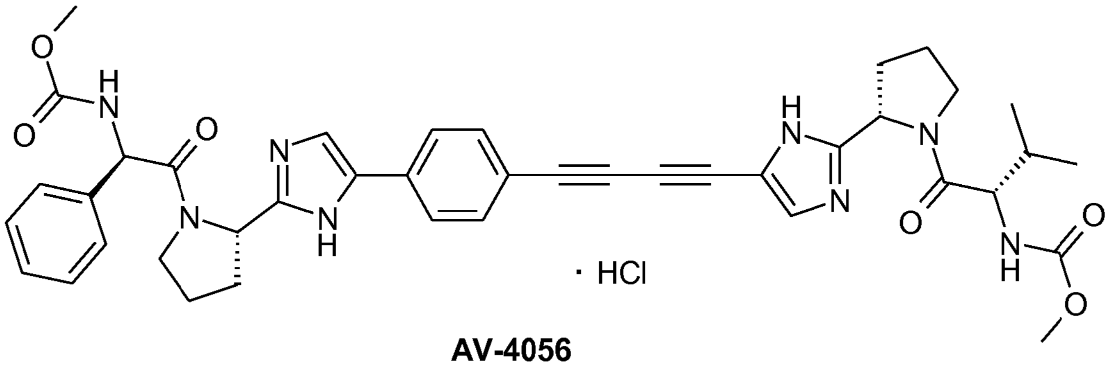

**AV-4056**

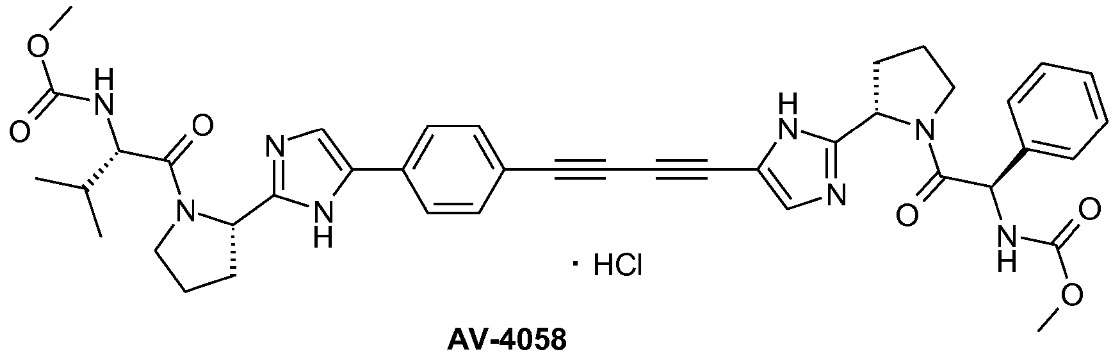

**AV-4058**

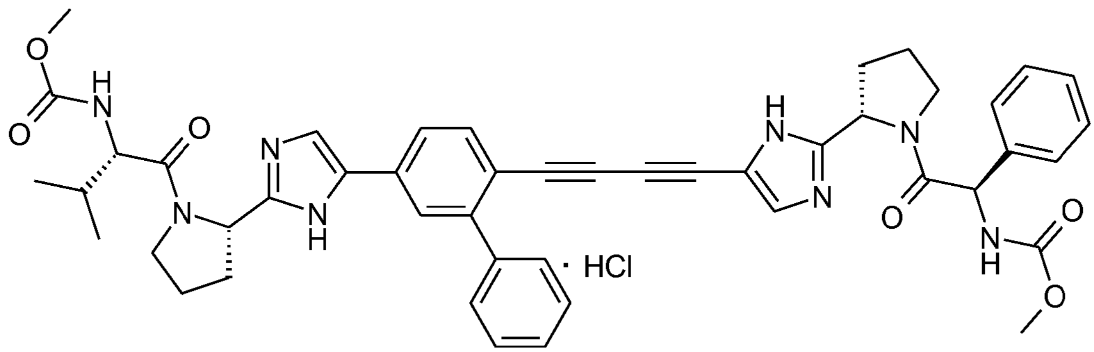

**AV-4067**

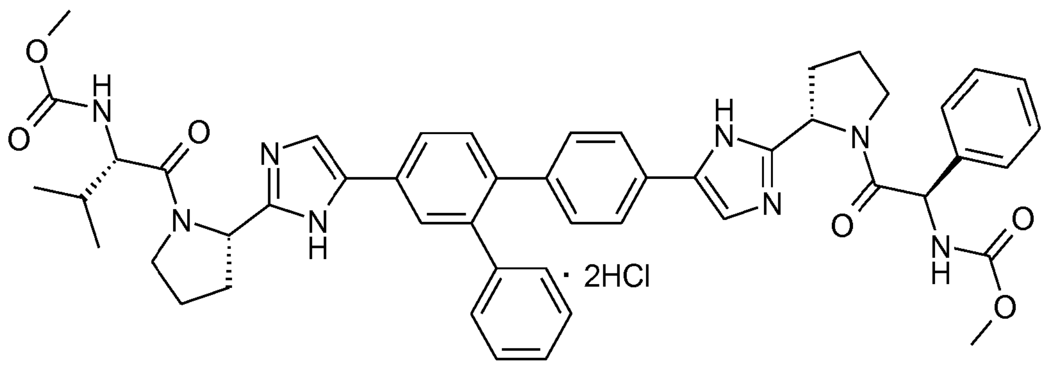

**AV-4084**

Samatasvir (IDX 18719, IDX 719) [Patent WO 2011075615, 2011. Bilello J. P. et al. In vitro activity and resistance profile of samatasvir, a novel NS5A replication inhibitor of hepatitis C virus. Antimicrobobial Agents Chemother. 2014, 58(8), 4431-4442.],

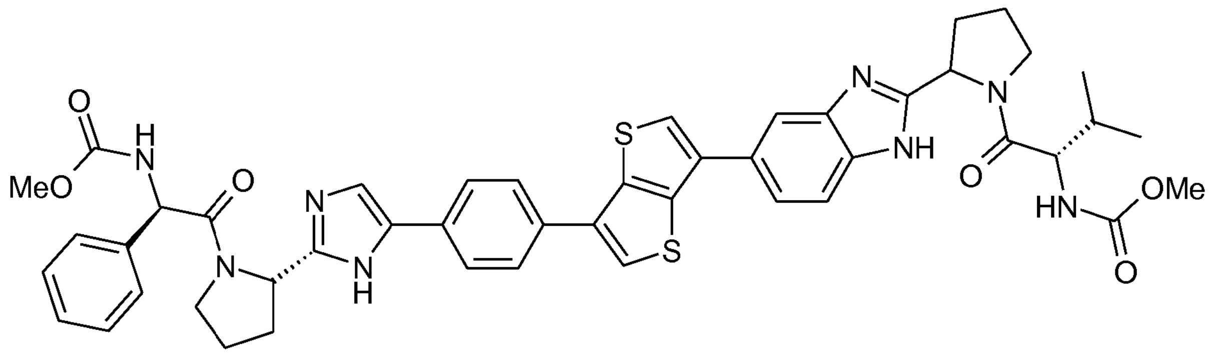

**Samatasvir (IDX 18719, IDX 719)**

Odalasvir (ACH-3102) [Nakamoto S. et al. Hepatitis C virus NS5A inhibitors and drug resistance mutations. World J. Gastroenterol. 2014, 20(11), 2902-2912.].

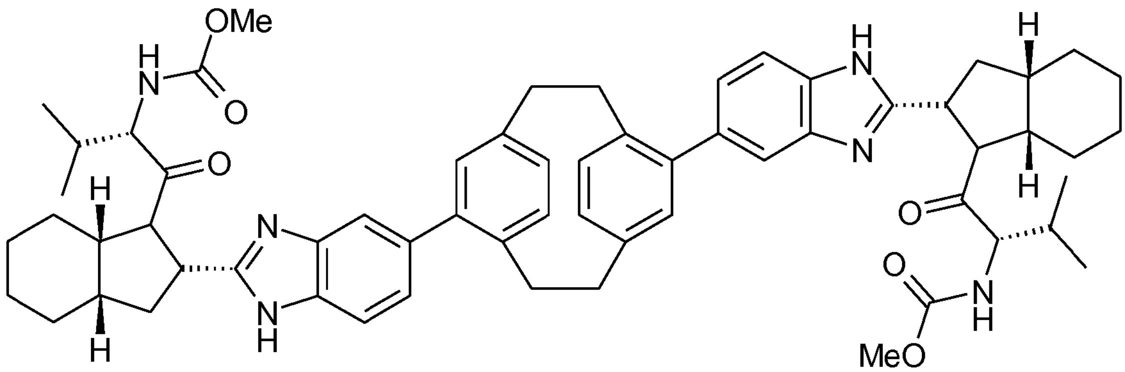

**Odalasvir (ACH-3102)**

Combinations of NS5A and NS5B inhibitors make it possible not only to avoid the use of interferon and ribavirin in the treatment of hepatitis C, but also to significantly improve the efficacy of treatment. For example, clinical trials of the combination drug Epclusa (one tablet of Epclusa contains 400 mg of sofosbuvir and 100 mg of velpatasvir) have demonstrated high potency (SVR12 ≥ 95%) in subjects without cirrhosis and in subjects with compensated cirrhosis, genotypes 1, 2, 3, 4, 5 and 6 of HCV infection in adults [http://www.gilead.com/~/media/files/pdfs/medicines/liver-disease/epclusa/epclusa_pi.pdf].

Protease inhibitors, too, have been successfully used in the combination therapy of hepatitis C, among them being Narlaprevir (SCH 900518), an inhibitor of the non-structured protein 3 HCV (HCV NS3) [Arasappan A. et al. Discovery of Narlaprevir (SCH 900518): A Potent, Second Generation HCV NS3 Serine Protease Inhibitor. ACS Med. Chem. Lett., 2010, 1 (2), 64-69], and Simeprevir (Olysio), an inhibitor of non-structured protein HCV NS3/NS4 (HCV NS3/4A) [https://www.tga.gov.au/ sites/default/files/auspar-simeprevir-141027-pi.docx].

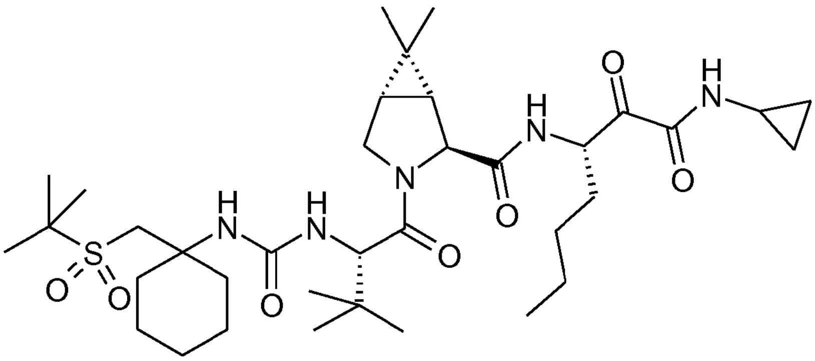

**Narlaprevir (SCH 900518)**

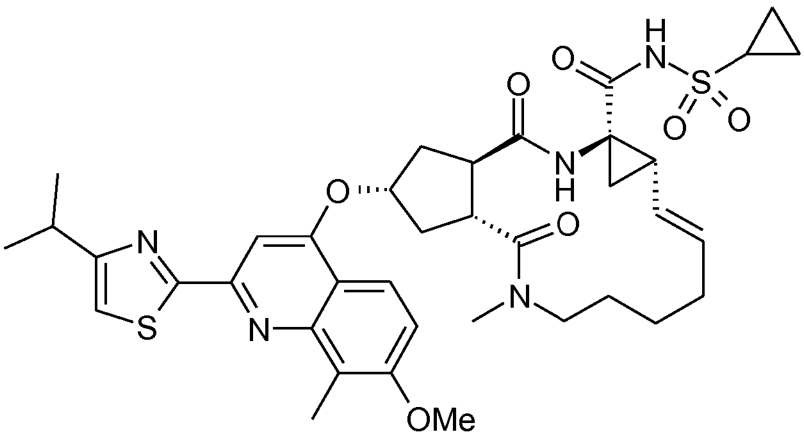

**Simeprevir (Olysio)**

Recently, an antiviral composition has been proposed, wherein, the phosphoramidate nucleotide PSI-7851, which is isopropyl (S)-2-{[(2R,3R,4R,5R)-5-(2,4-dioxo-3,4-dihydro-2H-pyrimidin-1-yl)-4-fluoro-3-hydroxy-4-methyltetrahydrofuran-2-ylmethoxy]-phenoxy-phosphorylamino}-propionate or its phosphoric stereoisomers (*R*p isomer: PSI-7976 and Sp isomer: PSI-7977), is used as the prodrug of an HCV NS5B polymerase inhibitor [US Pat. 7964580 (2011), RU 2478104 (2013)].

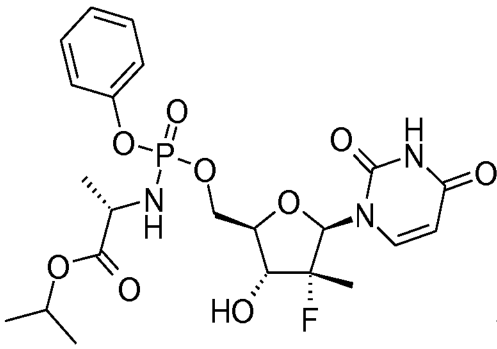

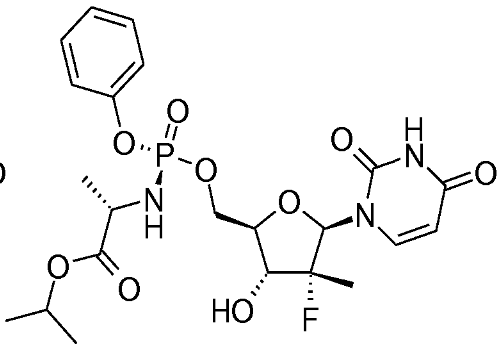

**PSI-7851** **$R_p$ isomer: PSI-7976**

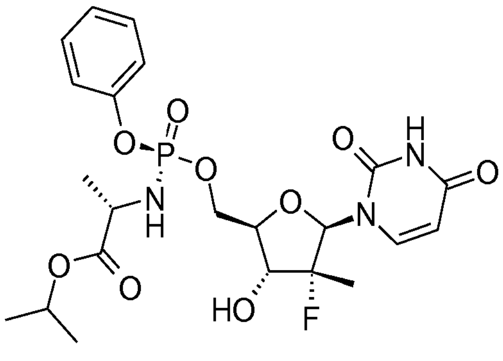

***S*p isomer: PSI-7977 (Sovaldi)**

An antiviral composition comprising PSI-7977 known as Sovaldi® or Sofosbuvir http://www.gilead.com/~/media/Files/ pdfs/medicines/ liver-disease/sovaldi/sovaldi_pi.pdf] is currently widely used for the combination treatment of hepatitis C, including in combination with NS5A HCV, NS3, NS3/4 inhibitors and other antiviral components. PSI-7851 and its stereoisomers PSI-7976 and PSI-7977 metabolize to triphosphate PSI-7409, which actually is an HCV NS5B polymerase inhibitor [E. Murakami et al. Mechanism of activation of PSI-7851 and its diastereoisomer PSI-7977. J. Biol. Chem. 2010, 285(45), 34337-34347].

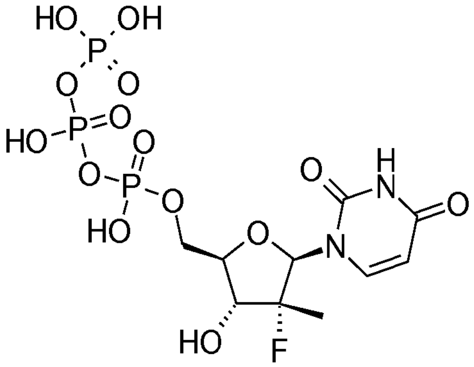

**PSI-7409**

The drug Sovaldi® [M. J. Sofia et al. Discovery of a β-D-20-Deoxy-20-r-fluoro-20-β-C-methyluridine Nucleotide Prodrug (PSI-7977) for the Treatment of Hepatitis C Virus. J. Med. Chem. 2010, 53, 7202-7218. M. J. Sofia et al. Nucleoside phosphoramidate prodrugs. US 7964580 (2011).] has become the first nucleotide approved by both FDA and EC for the combination therapy of hepatitis C patients infected with various genotypes (gT) of the hepatitis C virus. Clinical trials have demonstrated its high efficiency against six HCV genotypes (gT1-gT6) [I.M. Jacobson et al. Sofosbuvir for hepatitis C genotype 2 or 3 in patients without treatment options. Engl. J. Med. 2013, 368, 1867-1877. E. Lewirz et al. Sofosbuvir for previously untreated chronic hepatitis C infection. Engl. J. Med. 2013, 368, 1878-1887].

However, despite recent progress in the treatment of hepatitis C, a quest for novel antiviral compositions comprising a prodrug of HCV NS5B inhibitors with a more effective metabolism into triphosphate PSI-7409 and improved properties remains a major challenge.

**Summary of the invention**

The inventors have surprisingly found that antiviral compositions comprising, as a prodrug of HCV NS5B polymerase inhibitor, a previously unknown compound of general formula 1 or its phosphorus stereoisomer of formulas 1.1 or 1.2 (Sp stereoisomer: 1.1 and Rp stereoisomer: 1.2) are potent antiviral agents that can be used for the treatment of hepatitis C

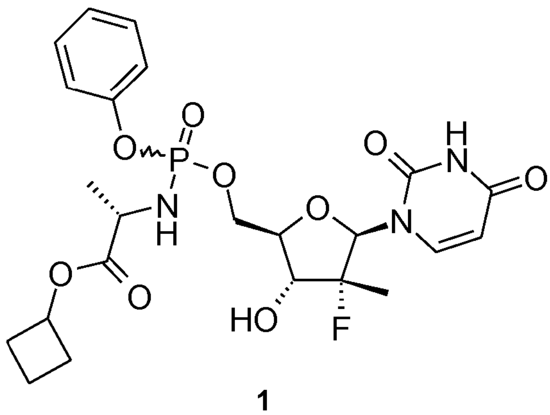

**1**

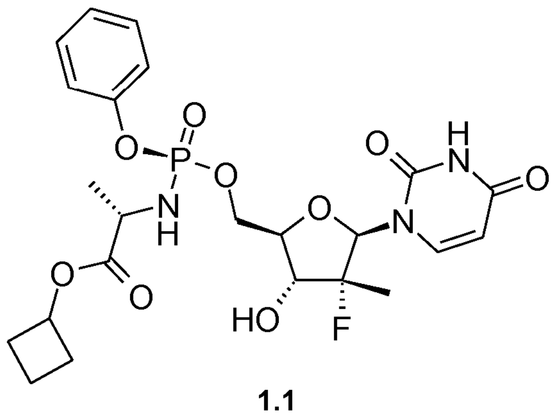

**1.1**

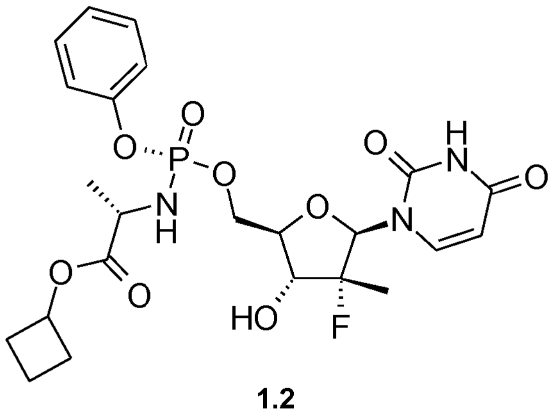

**1.2**

The inventors have found that the total concentration of triphosphate PSI-7409 resulting from metabolism in rat liver during the entire period of observation of a previously unknown prodrug of cyclobutyl (*S*)-2-{(*S*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydro-furan-2-ylmethoxy]-phenoxy-phosphorylamino}-propanoate (1.1) is $AUC_{0-t}$ = 27079 ng.h/g, which is 1.6 times higher than the total concentration of PSI-7409 ($AUC_{0-t}$ = 16796 ng·h/g) resulting from Sovaldi® metabolism in rat liver [M. J. Sofia et al. Discovery of a β-D-20-Deoxy-20-r-fluoro-20-β-C-methyluridine Nucleotide Prodrug (PSI-7977) for the Treatment of Hepatitis C Virus. J. Med. Chem. 2010, 53, 7202-7218].

It is to be noted that the combination treatment of hepatitis C currently involves the use of an antiviral composition in the form of tablets comprising 400 mg of Sovaldi®, which is the most effective daily dose The novel antiviral composition is more potent for hepatitis C therapy, since it comprises an active ingredient, in particular, the prodrug of formula 1.1, which more effectively metabolizes to triphosphate PSI-7409 in mammalian liver and which actually is an HCV NS5B polymerase inhibitor. This makes it possible to reduce the daily dose of the prodrug of formula 1.1 as compared to that of Sovaldi® thus reducing side effects.

The above data convincingly prove the novelty and the inventive level (efficacy) of the present invention.

Listed below are definitions of various terms used to describe this invention. These definitions apply to the terms as they are used throughout this specification and claims, unless otherwise limited in specific instances, either individually or as part of a larger group.

The term "active component" (drug substance) refers to a physiologically active compound of synthetic or other (biotechnological, plant, animal, bacterial, and so on) origins, which exhibits pharmacological activity and is an active ingredient of a pharmaceutical composition.

The term "crystalline form" refers to a substance structure wherein the molecules are arranged to form a crystal lattice.

The term "polycrystalline form" refers to a polycrystalline substance structure consisting of a plurality of monocrystals, or crystallites of certain crystalline form.

The term "medicinal drug" refers to a compound (or a mixture of compounds forming a pharmaceutical composition) in the form of tablets, capsules, injections, ointments, or other finished dosage forms intended for the restoration, improvement, or modification of physiological functions in humans and animals, and for the treatment and prophylaxis of diseases, for diagnostics, anesthesia, contraception, cosmetology, etc.

The term "pharmaceutical composition" refers to a composition comprising the compound of general formula 1 and at least one of the components selected from the group consisting of pharmaceutically acceptable and pharmacologically compatible fillers, solvents, diluents, carriers, excipients, distributing, and receptive agents, delivery agents such as preservatives, stabilizers, fillers, disintegrators, moisteners, emulsifiers, suspending agents, thickeners, sweeteners, flavoring agents, aromatizing agents, antibacterial agents, fungicides, lubricants, and prolonged delivery controllers, the choice and proportions of which depend on the nature and route of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof.

Protection against microorganisms can be provided using various antibacterial and antifungal agents, such as parabens, chlorobutanol, sorbic acid, and the like. Said composition may also include isotonic agents, such as sugar, sodium chloride, and the like. The sustained action of the composition can be achieved using agents that decelerate the absorption of the active ingredient, for example, aluminum monostearate and gelatin. Examples of suitable carriers, solvents, diluents and delivery agents include water, ethanol, polyalcohols and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and distributors are starch, alginic acid and salts thereof, and silicates. Examples of lubricants are magnesium stearate, sodium lauryl sulfate, talc, and polyethylene glycol of high molecular weight. A pharmaceutical composition for peroral, sublingval, transdermal, intramuscular, intravenous, subcutaneous, and local or rectal administration of the active ingredient, alone or in combination with another active compound, may be administered to animals and people in a standard administration form as a mixture with traditional pharmaceutical carriers. Suitable standard administration forms include peroral forms, such as tablets, gelatin capsules, pills, powders, granules, chewing gums, and peroral solutions or suspensions; sublingval and transbuccal administration forms; aerosols; implants; local, transdermal, subcutaneous, intramuscular, intravenous, intranasal, or intraocular forms; and rectal administration forms.

The term "inert filler" as used herein refers to a compound that is used for forming a pharmaceutical composition and is, as a rule, safe, nontoxic, and neither biologically nor otherwise undesirable, and comprises excipients acceptable for veterinary and human pharmaceutical use. Compounds of this invention may be administered individually but are generally administered in a mixture with one or more pharmaceutically acceptable excipients, diluents, or carriers chosen depending on the contemplated route of drug administration and standard pharmaceutical practice.

The term "therapeutically effective amount," as used herein, refers to an amount of a substance, prodrug, or drug needed for alleviating the symptoms of the disease in the subject. The dose of a substance, prodrug, or drug will meet individual demands in each particular case. Said dose may vary in a wide range depending on numerous factors like the severity of the disease to be treated, the age and the general condition of the patient, other medicaments used for the patient's treatment, the mode and route of administration, and the experience of the attending doctor. For oral administration, the daily dose is approximately 0.01-10 g, including all values therebetween, both in monotherapy and/or combination therapy. The preferred daily dose is around 0.1-7 g. As a rule, in order to quickly alleviate or eliminate the virus, a higher loading dose is given at the beginning of treatment with a subsequent reduction of the dose to a level sufficient to prevent an infection burst.

The term "subject" refers to a mammal including, but not limited to, cattle, hogs, sheep, chickens, turkeys, buffalos, lamas, ostriches, dogs, cats, and humans; a human subject is most preferable. It is assumed that a subject's treatment may involve the use of any prodrug of general formula 1 and its stereomers, isotopically enriched forms thereof, pharmaceutically acceptable salts, hydrates, solvates, and crystalline or polymorphic forms or their combinations with another compound, including with an HCV NS5A inhibitor.

The subject matter of the present invention is an antiviral pharmaceutical composition for a combination treatment of hepatitis C virus in a subject in need thereof, said composition comprising an effective amount of a compound selected from cyclobutyl (*S*)-2-{[(2*R*,3*R*,4*R*,5*R*)-5-(2,4-dioxo-3,4-dihydro-2H-pyrimidin-1-yl)-4-fluoro-3-hydroxy-4-methyl-tetrahydrofuran-2-ylmethoxy]-phenoxy-phosphorylamino}-propanoate of general formula 1, and/or cyclobutyl (S)-2-{(S)-[(2R,3R,4R,5R)-5-(3,4-dihydro-2,4-dioxo-2H-pyrimidin-1-yl)-3-hydroxy-4-methyl-4-fluoro-tetrahydro-furan-2-ylmethoxy]-phenoxy-phosphorylamino}-propanoate of formula 1.1, and/or cyclobutyl (S)-2-{(R)-[(2R,3R,4R,5R)-5-(3,4-dihydro-2,4-dioxo-2H-pyrimidin-1-yl)-3-hydroxy-4-methyl-4-fluoro-tetrahydro-furan-2-ylmethoxy]-phenoxy-phosphorylamino}-propanoate of formula 1.2, or an isotopically enriched, crystalline or polycrystalline form thereof

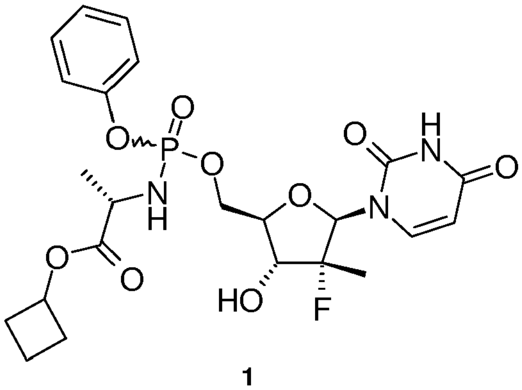

1

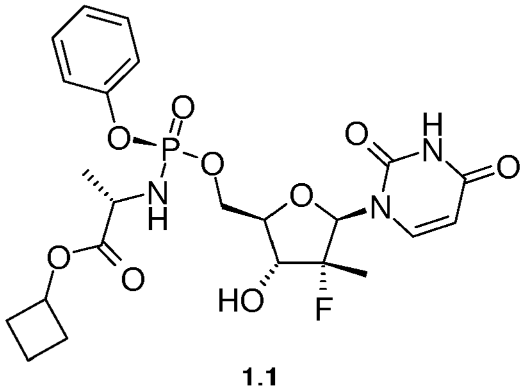

1.1

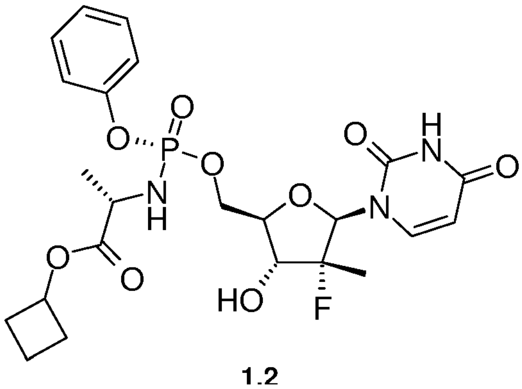

1.2

and a pharmaceutically acceptable carrier.

Preferable the antiviral pharmaceutical composition contains, as a prodrug of HCV NS5B polymerase inhibitor, cyclobutyl (S)-2-{(S)-[(2R,3R,4R,5R)-5-(3,4-dihydro-2,4-dioxo-2*H*-pyrimidin-1-yl)-3-hydroxy-4-methyl-4-fluoro-tetrahydro-furan-2-ylmethoxy]-phenoxy-phosphorylamino}-propanoate of formula 1.1, an isotopically enriched, crystalline or polycrystalline form thereof.

As shown above, the novel antiviral pharmaceutical composition is more potent than known compositions including one comprising Sovaldi® as an HCV NS5B polymerase inhibitor.

The subject matter of the present invention is an antiviral pharmaceutical composition for the treatment of hepatitis C virus in mammals, said composition comprising the prodrug of general formula 1 or its stereoisomer, isotopically enriched, crystalline or polycrystalline forms optionally in combination with pharmaceutically acceptable filler, carrier, additive, or diluent.

Said antiviral pharmaceutical composition may be prepared in a variety of peroral dosage forms and carriers including tablets, film-coated tablets, hard and soft gelatin capsules, solutions, emulsions, syrups, or suspensions. The antiviral pharmaceutical composition of this invention is effective when administered in the form of suppositories. Generally, the most convenient route of administration is peroral using a common daily dosage regimen that can be adjusted depending on the severity of disease and a patient's antiviral or antitumor drug reaction.

Said antiviral pharmaceutical composition with one or more common excipients, carriers, or diluents may be prepared as pharmaceutical compositions and single-unit dosage forms thereof. Pharmaceutical compositions and unit dosage forms may consist of ordinary ingredients in usual proportions with or without additional active compounds and dosage forms. Said antiviral composition may comprise any appropriate effective amount of active ingredient depending on the prescribed daily dose. The antiviral composition may be used in the form of solid substances, such as tablets or filled capsules; in the form of semisolid powders, agents with sustained release or liquids such as suspensions, emulsions, or filled capsules for peroral administration; or in the form of suppositories for rectal or vaginal administration. A typical medication will comprise approximately from 5wt% to 95wt% of the active compound or the compound. The terms "medication" or "dosage form" are meant to include both solid and liquid compositions of active compound, so that it would be clear for a person skilled in the art that the active ingredient may exist in the form of different medications depending on the dose required and pharmacokinetic parameters.

Solid dosage forms include powders, tablets, pills, capsules, suppositories, and dispersible granules. A solid carrier refers to one or more substances that can act as diluents, flavors, solubilizers, lubricants, suspending agents, binding agents, preservatives, disintegrants, or encapsulating material. A powder carrier is generally a fine-grained solid

mixed with a fine-grained active component. In the tablets, the active component is usually mixed, in appropriate proportions, with a carrier having a necessary binding capacity and compacted into the desired shape and size. Suitable carriers include, but are not limited to, magnesium carbonate, magnesium stearate, talc, sugar, lactose, pectin, dextrin, starch, gelatin, tragacant, methylcellulose, sodium carboxymethylcellulose, low-melting wax, cocoa butter, and the like. Solid preparations may, in addition to the active ingredient, comprise dyes, flavoring agents, stabilizers, buffers, artificial and natural sweeteners, dispersants, thickeners, solubilizers, and the like.

Liquid compositions are suitable for peroral administration too. Liquid dosage forms include emulsions, syrups, elixirs, and aqueous suspensions. They comprise solid drug forms to be converted to liquid medications immediately before use. Emulsions may be prepared in solutions, for example, in aqueous solutions of propylene glycol, or they may comprise emulsifiers, such as lecithin, sorbitol monooleate, or gum arabic. Aqueous suspensions may be prepared by dispersing a fine-grained active ingredient in water with ductile materials, such as natural or synthetic gums, resins, methylcellulose, sodium carboxymethylcellulose, and other well-known suspending agents.

The antiviral pharmaceutical composition may be prepared for administration in the form of suppositories. Low-melting wax, such as a mixture of glycerides of fatty acids or cocoa butter, is first melt and then the active ingredient is homogeneously dispersed by, for example, stirring. The molten homogeneous mixture is poured into moulds of a suitable size and allowed to cool and solidify.

Antiviral pharmaceutical composition may be prepared for vaginal administration. It will be appropriate to apply suppositories, tampons, creams, gels, pastes, foams, or sprays comprising, in addition to the active ingredient, carriers that are well known in the art.

More preferable is an antiviral pharmaceutical composition, which, along with the novel prodrug of general formula 1, or its stereoisomer of formula 1.1, isotopically enriched, crystalline or polycrystalline forms, additionally comprises a therapeutically effective amount of an antiviral or anticancer agent.

Preferable is an antiviral pharmaceutical composition, which, along with the novel prodrug of general formula 1, or its stereoisomer of formula 1.1, isotopically enriched, crystalline or polycrystalline forms, additionally comprises a therapeutically effective amount of an HCV NS5A inhibitor selected from the group including: Daclatasvir (Daklinza, BMS790052), Hepavivir (AV-4025), AV-4056 and AV-4058, Ombitasvir (ABT-267), Elbasvir (MK-8742), or Velpatasvir (VEL, GS-5816), or Narlaprevir (SCH 900518)-an HCV NS3 inhibitor, or Simeprevir (Olysio)-an HCV NS3/NS4 inhibitor.

Also preferable is an antiviral pharmaceutical composition, which, along with the novel prodrug of general formula 1, or its stereoisomer of formula 1.1, isotopically enriched, crystalline or polycrystalline forms, additionally comprises a therapeutically effective amount of an HBV DNA polymerase inhibitor and a HIV 1 reverse transcriptase (RT) inhibitor.

A further subject matter of the present invention is a combination of active agents comprising, a compound selected from cyclobutyl (*S*)-2-{[(2*R*,3*R*,4*R*,5*R*)-5-(2,4-dioxo-3,4-dihydro-2H-pyrimidin-1-yl)-4-fluoro-3-hydroxy-4-methyl-tetrahydrofuran-2-ylmethoxy]-phenoxy-phosphorylamino}-propanoate of general formula 1, and/or its phosphorus stereoisomer (*S*p stereoisomer of formula 1.1 or *R*p stereoisomer of formula 1.2), or an isotopically enriched, crystalline or polycrystalline forms thereof, and s one or more other antiviral or anticancer agents for use in combination therapy of hepatitis C. Preferably the combination therapy the combination therapy of hepatitis C in a subject in need thereof includessimultaneous or successive administration of a therapeutically effective amount of the agents. It is understood that there may be any time span between the successive administrations of agents.

Examples of "other antiviral agents" include, but are not limited to, HCV NS3 protease inhibitors [US 20140296136, US 8,987,195, US 7973040, US 2012214783], HCV NS4 inhibitors [EP1497282], HCV NS3/NS4 inhibitors [EP 2364984], and HCV NS5A inhibitors [C. Wang et al. Hepatitis C virus RNA elimination and development of resistance in replicon cells treated with BMS-790052. Antimicrob. Agents Chemother. 2012, 56, 1350-1358. https://en.wikipedia.org/wiki/Daclatasvir; A.V. Ivachtchenko et al. Discovery of Novel Highly Potent Hepatitis C Virus NS5A Inhibitor (AV4025). J. Med. Chem. 2014, 57, 7716-7730; Pat. Appl. US 14/845,333); Toll-like receptor agonists (see WO 2015023958, WO 2012097012); and other inhibitors (see WO 2014106019, WO 2014033176, WO 2014033170, WO 2014033167, WO 2013006394, US 20090163545].

It is assumed that the other antiviral agent is, but is not limited to, interferon alpha, interferon beta, pegylated interferon alpha, ribavirin, levovirin, viramidine, another nucleoside HCV polymerase inhibitor, non-nucleoside HCV polymerase inhibitor, HCV protease inhibitor, HCV helicase inhibitor or HCV fusion inhibitor, HBV DNA polymerase inhibitor, and HIV 1 reverse transcriptase (RT) inhibitor. When the prodrug of general formula 1 or its stereoisomer, isotopically enriched analogs, or crystalline or polycrystalline forms are administered in combination with another antiviral or anticancer agent, the activity may be increased against the initial activity of the prodrug. In combination therapy, the administration of agents may be simultaneous or successive regarding the prodrug of general formula 1 or its stereoisomers, isotopically enriched analogs, or crystalline or polycrystalline forms. The notion "simultaneous administration" as used herein thus means administration of agents at the same time or at different times. The administration of two or more agents at the same time may be performed by using one preparative form comprising two or more active ingredients or, in essence, by simultaneously administering two or more dosage forms with one active agent. It should be understood

that any reference to therapy as used herein covers prophylaxis as well. In addition, the term "therapy" of viral infection, as used herein, includes treatment or prophylaxis of a disease or a condition associated with a mediated viral infection, or clinical symptoms thereof.

The inventors have surprisingly found that the novel antiviral pharmaceutical composition comprising the prodrug of general formula 1 and/or its stereoisomer of formula 1.1 is more effective than other known antiviral compositions and, in particular, more potent than one comprising Sovaldi®.

Thus, Sovaldi® has against HCV genotype 1b (gT1b) $EC_{50}$ = 0.045-0.170 μM [http://www.hcvdruginfo.ca/downloads/HCV_Sofosbuvir.pdf] and $EC_{90}$ = 0.59 μM, while the novel prodrug of formula 1.1 has $EC_{50}$ = 15.0-27.0 nM and $EC_{90}$ = 128.0 nM (Table 2), which means that the novel prodrug of formula 1.1 is more than three times more active than Sovaldi®. The half-life of the prodrug of formula 1.1 in human liver microsomal S9 fraction is $T_{1/2}^{hS9}$ = 0.05 h, while Sovaldi® has $T_{1/2}^{hS9}$ = 0.57 h (Table 3), which means that the metabolic rate of the novel prodrug of formula 1.1 in human liver microsomal S9 fraction is 11 times faster than that of Sovaldi®. In addition, the concentration and $AUC_{24h}$ triphosphate PSI-7409 in the rat liver resulting from the metabolic process of the prodrug of formula 1.1 are Cmax =3 224.0 ng/g and $AUC_{24h}$ = 30 487.0 ngh/g, respectively, while Sovaldi's similar metabolism leads to Cmax = 1 934.0 ng/g and $AUC_{24h}$ = 16 796.0 ng.h/g (Table 4). This testifies to the fact that the novel prodrug metabolizes into requisite triphosphate PSI-7409 (drug) in the liver almost two times more effectively.

**Best embodiment**

The present invention will now be described in terms of certain embodiments which are not intended to limit its scope. On the contrary, the present invention covers all alternatives, modifications, and equivalents that can be included within the scope of the claims. Thus, the following examples, which include specific embodiments, will illustrate this invention without limiting it.

**Example 1.**

Synthetic protocol for the prodrug cyclobutyl (*S*)-2-{[(2*R*,3*R*,4*R*,5*R*)-5-(3,4-dihydro-2,4-dioxo-2*H*-pyrimidin-1-yl)-3-hydroxy-4-methyl-4-fluoro-tetrahydro-furan-2-ylmethoxy]-phenoxy-phosphorylamino}-propanoate of general formula (1) and its stereoisomers of formula 1.1 and formula 1.2 (Scheme 1).

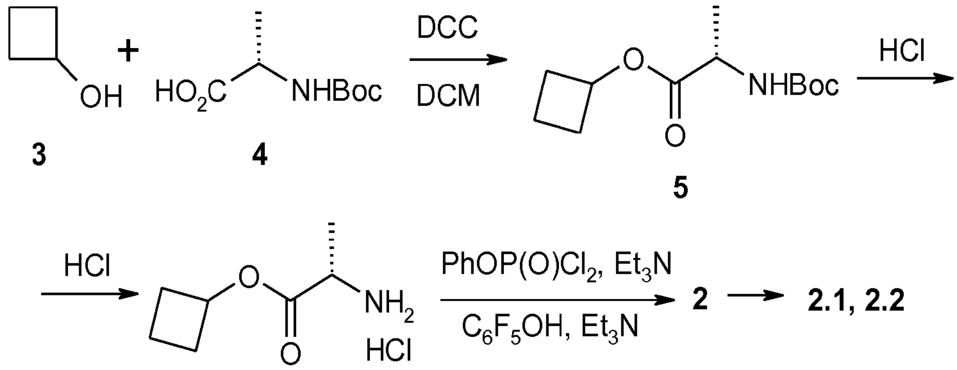

3 4 5

6

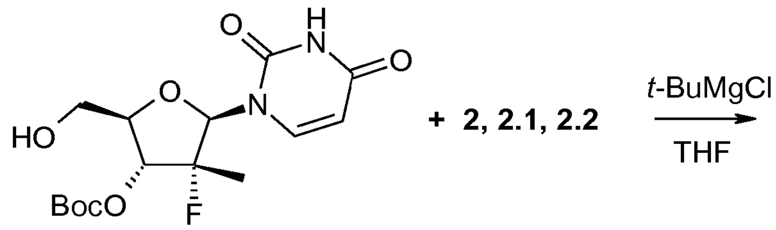


7

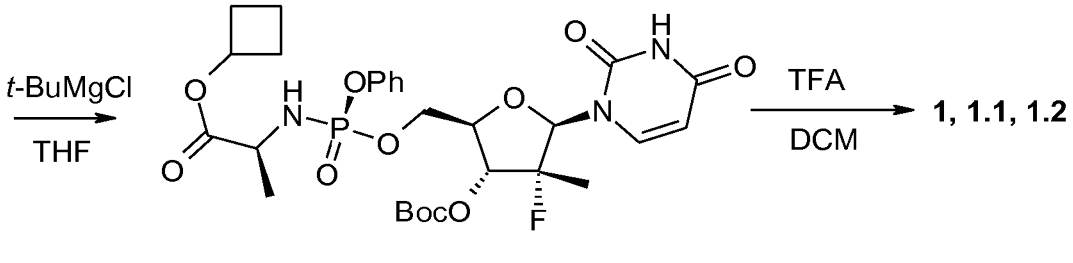


8, 8.1, 8.2

To a solution of *N*-Boc-L-alanine (4: 15.5 g, 81.9 mmol) in dichloromethane (300 ml), DCC (16.9 g, 81.9 mmol) was added at 0°C and 5 min later, cyclobutanol (3: 5.6 g, 78.0 mmol) and DMAP (2.0 g, 16.4 mmol). The mixture was stirred overnight evaporated *in vacuum*, and the residue was treated with ethyl acetate (300 ml). The residue was filtered off and washed with ethyl acetate. The filtrate was washed with a 5% solution of citric acid (2 x 100 ml), a saturated $NaHCO_3$ solution (2 x 100 ml), and brine, dried over $Na_2SO_4$, and evaporated *in vacuum* to afford 19.6 g (98 %) of (S)-cyclobutyl 2-(*tert*-butoxycarbonylamino)-propanoate (5) as a white powder. $^1H$ NMR (400 MHz, DMSO-$d_6$) δ 7.22 (d, *J* = 7.2 Hz, 0.85H), 6.87 (m, 0.15H), 4.89 (p, *J* = 7.2 Hz, 1H), 3.94 (m, 1H), 2.26 (m, 2H), 1.98 (m, 2H), 1.74 (m, 1H), 1.59 (m, 1H), 1.38 (s, 7.5H), 1.34 (brs, 1.5H), 1.22 (d, *J* = 7.2Hz, 3H).

To a solution of compound 5 (19.6 g, 80.6 mmol) in dioxane (50 ml), HCl (230 ml, 3M) in dioxane was added, the mixture was allowed to stir overnight and evaporated *in vacuum* . The residue was ntreated with ether (400 ml) and stirred overnight. The residue was filtered off The residue was filtered off, washed with ether, and dried *in vacuum* to afford 14.1 g (97 %) of (S)-cyclobutyl 2-amino-propanoate hydrochloride (6) as a white powder. $^1H$ NMR (400 MHz, DMSO-$d_6$) δ 8.56 (brs, 3H), 5.00 (p, *J* = 7.6 Hz, 1H), 4.02 (q, *J* = 7.2 Hz, 1H), 2.31 (m, 2H), 2.07 (m, 2H), 1.78 (m, 1H), 1.62 (m, 1H), 1.41 (d, *J* = 7.2 Hz, 3H).

Phenyl dichlorophosphate (16.9 g, 80.2 mmol) was added to a solution of compound 6 (14.4 g, 80.2 mmol) in dichloromethane (214 ml). The mixture was cooled to -75-70°C, and a solution of triethylamine (16.2 g, 160.4 mmol) in dichloromethane (16 ml) was added dropwise while maintaining the temperature at -75-70 °C. The mixture was allowed to stir at -70°C for 30 min and then heated to -20 °C. A solution of pentafluorophenol (14.6 g, 79.4 mmol) in dichloromethane (105 ml) was added at -20-10 °C, then a solution of triethylamine (8.1 g, 80.2 mmol) in dichloromethane (8 ml) was added dropwise, and the mixture was left to stir overnight at room temperature. The mixture was evaporated in vacuum, ethyl acetate (500 ml) and water (500 ml) were added, the organic layer was separated, washed with 5% $NaHCO_3$ solution, brine, dried over $Na_2SO_4$ and evaporated in vacuum. To the residue 200 ml of 6:1 hexane/ethyl acetate mixture was added and stirred overnight. The resulting residue was filtered off, washed with a 6:1 hexane/ethyl acetate mixture (50 ml), and air-dried to afford 16.7 g of cyclobutyl (S)-2-((perfluorophenoxy)(phenoxy)phosphorylamino)-propanoate (2).

The resulting product (2) was recrystallized from a hexane/ethyl acetate (4:1) mixture (500 ml) to afford 13.8 g (37%) of cyclobutyl (*S*)-2-((*S*)-(perfluorophenoxy)-(phenoxy)-phosphorylamino)propanoate of formula (2.1) as a white loose powder. $^1H$ NMR (300 MHz, DMSO-$d_6$) δ 7.42 (m, 2H), 7.24 (m, 3H), 6.87 (dd, $J_1$ = 14.1 Hz, $J_2$ = 10.2 Hz, 1H), 4.87 (p, *J* = 7.5 Hz, 1H), 3.94 (m, 1H), 2.23 (m, 2H), 1.94 (m, 2H), 1.71 (m, 1H), 1.58 (m, 1H), 1.27 (d, *J* = 7.2 Hz, 3H). The generic solution left after washing with a mixture of hexane/ethyl acetate (6:1) during recrystallization of the compound of formula 2.1 was evaporated in vacuum and thrice recrystallized from hexane to afford cyclobutyl (*S*)-2-((*R*)-(perfluorophenoxy)-(phenoxy)-phosphorylamino)-propanoate (2.2) as a white loose powder. $^1H$ NMR (300 MHz, DMSO-$d_6$) δ 7.42 (m, 2H), 7.26 (m, 3H), 6.85 (dd, $J_1$ = 13.8 Hz, $J_2$ = 10.2 Hz, 1H), 4.88 (p, *J* = 7.5 Hz, 1H), 3.95 (m, 1H), 2.24 (m, 2H), 1.93 (m, 2H), 1.72 (m, 1H), 1.59 (m, 1H), 1.28 (d, *J* = 6.9 Hz, 3H).

To a solution of *tert*-butyl (2*R*,3*R*,4*R*,5*R*)-5-(2,4-dioxo-3,4-dihydropyrimidin-1(2*H*)-yl)-2-(hydroxymethyl)-4-methyl-4-fluoro-tetrahydrofuran-3-yl carbonate (7: 5 g, 13.9 mmol) in THF (165 ml), a 1M solution of *tert*-butylmagnezium chloride in THF (31.3 ml, 31.3 mmol) was added under argon at 0°C, and the mixture was stirred for 30 min at room

temperature. A solution of the compound of formula 2.1 (7.8 g, 16/7 mmol) in THF (30 ml) was added at 0-5°C using a syringe, and the mixture was stirred under argon for 24 hours. Upon addition of methanol (10 ml), the mixture was evaporated in vacuum. The residue was dissolved in 500 ml of ethyl acetate, washed with 5% solution of citric acid, 5% solution of $NaHCO_3$, dried over $Na_2SO_4$, and evaporated in vacuum. The residue was chromatographed on silica gel using hexane/ethyl acetate (1:2) as an eluent to afford 5.89 g (66 %) of cyclobutyl (*S*)-2-((*S*)-(((2*R*,3*R*,4*R*,5*R*)-3-(*tert*-butoxycarbonyloxy)-5-(2,4-dioxo-3,4-dihydropyrimidin-1(2*H*)-yl)-4-methyl-4-fluoro-tetrahydrofuran-2-yl)methoxy)(phenoxy)phosphorylamino)propanoate (8.1) looking like a colorless solidified foam. LC-MS (ESI) 642 $(M+H)^+$.

Similarly, cyclobutyl (*S*)-2-((((2*R*,3*R*,4*R*,5*R*)-3-(*tert*-butoxycarbonyloxy)-5-(2,4-dioxo-3,4-dihydropyrimidin-1(2*H*)-yl)-4-methyl-4-fluoro-tetrahydrofuran-2-yl)methoxy)(phenoxy)phosphorylamino)propanoate (8) was prepared starting from intermediates 7 and 2 (yield: 52%, LC-MS (ESI) 642 $(M+H)^+$) and cyclobutyl (*S*)-2-((*R*)-(((2*R*,3*R*,4*R*,5*R*)-3-(*tert*-butoxycarbonyloxy)-5-(2,4-dioxo-3,4-dihydropyrimidin-1(2*H*)-yl)-4-methyl-4-fluoro-tetrahydrofuran-2-yl)methoxy)(phenoxy)phosphorylamino)propanoate (8.2), starting from intermediates 7 and 2.2 (yield: 59%, LC-MS (ESI) 642 $(M+H)^{+}$).

To a solution of the compound of formula 8.1 (4.45 g, 6.9 mmol) in dichloromethane (60 ml), trifluoroacetic acid (60 ml) was added at 0°C. The mixture was stirred for 15 h at room temperature, evaporated in vacuum, dissolved in 250 mL of DCM, washed with 300 ml of 5% $NaHCO_3$, dried over $Na_2SO_4$, evaporated in vacuum, and recrystallized from a mixture of ethyl acetate and methyl-*tert*-butyl ether (1:1) to afford 2.7 g (71 %) of cyclobutyl (*S*)-2-((S)-(((2*R*,3*R*,4*R*,5*R*)-5-(2,4-dioxo-3,4-dihydropyrimidin-1(2*H*)-yl)-3-hydroxy-4-methyl-4-fluoro-tetrahydrofuran-2-yl)methoxy)-(phenoxy)-phosphorylamino)-propanoate of formula (1.1) as a white crystalline substance. LC-MS (ESI) 542 $(M+H)^+$. $^1$H NMR (400 MHz, DMSO-$d_6$) $\delta$ 11.51 (brs, 1H), 7.56 (d, $J$ = 8.0 Hz, 1H), 7.38 (m, 2H), 7.23 (m, 2H), 7.19 (m, 1H), 6.03 (m, 2H), 5.84 (d, $J$ = 6.8 Hz, 1H), 5.55 (dd, $J_1$ = 8.0 Hz, $J_2$ = 1.2 Hz, 1H), 4.85 (p, $J$ = 7.2 Hz, 1H), 4.37 (m, 1H), 4.27 (m, 1H), 4.01 (m, 1H), 3.83 (m, 2H), 2.23 (m, 2H), 1.95 (m, 2H), 1.71 (m, 1H), 1.56 (m, 1H), 1.25 (d, $J$ = 22.8 Hz, 3H), 1.23 (d, $J$ = 6.8 Hz, 3H).

Recrystallization of the prodrug of formula 1.1 from various solvents leads to polycrystalline or crystalline forms. Thus, recrystallization from a mixture of ethyl acetate with methyl-*tert*-butyl ether (1:1), ethanol, ethyl acetate, and a mixture of acetic acid with water yields prodrug 1.1 in polycrystalline forms essentially comprising an orthorhombic phase with unit cell parameters of $a$ = 28.1056(8)A, $b$ = 16.8998(4)A, and $c$ = 5.25380(12)A and a monoclinic phase with unit cell parameters of $a$ = 16.2770(6)A, $b$ = 16.9117(8)A, $c$ = 5.20429(15)A, and $\beta$ = 117.822(2)°.

Recrystallization of the prodrug of formula 1.1 from a mixture of dimethyl sulfoxide with water leands to a white crystalline substance consisting of an orthorhombic phase with unit cell parameters of $\boldsymbol{a}$ = 28.1056(8)A, $\boldsymbol{b}$ = 16.8998(4)A, and $\boldsymbol{c}$ = 5.25380(12)A.

Crystalline and polycrystalline forms have similar solubility values after recrystallization from various solvents at pH 2 and pH 7 varying from 0.18 to 0.25 mg/ml. The exception is a polycrystalline sample obtained from recrystallization from dimethyl sulfoxide, the solubility of which is a little higher and varies from 0.63 to 0.67 mg/ml (Table 1).

**Table 1.**

| Kinetic solubility of polycrystalline and crystalline forms of the prodrug of formula 1.1 for a wavelength of 260 nm | | | | | |
|---|---|---|---|---|---|
| Prodrug 1.1 recrystallized from | Form of prodrug 1.1 | Solubility, mg/ml | | | |
| | | at pH 2 | | at pH 7 | |
| | | value | SD | value | SD |
| Ethyl acetate and methyl-tert-butyl ether mixture | polycrystal | 0.25 | 0.00 | 0.24 | 0.001 |
| Ethanol | polycrystal | 0.20 | 0.00 | 0.20 | 0.003 |
| Ethyl acetate | polycrystal | 0.22 | 0.00 | 0.22 | 0.002 |
| Acetic acid | polycrystal | 0.63 | 0.009 | 0.67 | 0.041 |
| Dimethyl sulfoxide | crystal | 0.18 | 0.00 | 0.18 | 0.003 |

Similarly, compounds cyclobutyl (*S*)-2-((*R*)-(((2*R*,3*R*,4*R*,5*R*)-5-(2,4-dioxo-3,4-dihydropyrimidin-1(2*H*)-yl)-3-hydroxy-4-methyl-4-fluoro-tetrahydrofuran-2-yl)methoxy)-(phenoxy)-phosphorylamino)-propanoate (1) (yield: 58%; LC-MS (ESI) 542) and cyclobutyl (*S*)-2-((*R*)-(((2*R*,3*R*,4*R*,5*R*)-5-(2,4-dioxo-3,4-dihydropyrimidin-1(2*H*)-yl)-3-hydroxy-4-methyl-4-fluoro-tetrahydrofuran-2-yl)methoxy)-(phenoxy)-phosphorylamino)-propanoate (1.2) (yield: 64%; LC-MS (ESI) 542 $(M+H)^+$. $^1$H NMR (300 MHz, DMSO-$d_6$) $\delta$ 11.53 (brs, 1H), 7.56 (d, $J$ = 8.1 Hz, 1H), 7.38 (m, 2H), 7.19 (m, 3H), 6.08 (m, 2H), 5.91 (d, $J$ = 6.3 Hz, 1H), 5.57 (d, $J$ = 8.1 Hz, 1H), 4.85 (p, $J$ = 7.5 Hz, 1H), 4.41 (m, 1H), 4.27 (m, 1H), 4.05 (m,

1H), 3.79 (m, 2H), 2.23 (m, 2H), 1.94 (m, 2H), 1.70 (m, 1H), 1.56 (m, 1H), 1.24 (d, *J* = 23.4 Hz, 3H), 1.21 (d, *J* = 6.0 Hz, 3H)) were prepared.

**Example 2.**

Preparation of an antiviral composition in the form of tablet. Starch (500 mg), ground lactose (800 mg), talc (200 mg), and 1500 mg of the prodrug of formula 1.1 were mixed together and pressed into bar. The resulting bar was comminuted into granules and sifted through a sieve to collect granules of 14-16 mesh. The granules thus obtained were shaped into tablets of suitable form weighing 400 or 800 mg each.

**Example 3.**

Preparation of an antiviral composition in the form of capsules. The prodrug of formula 1.1 was carefully mixed with a lactose powder in a ratio of 1:1. The resulting powdery mixture was packed into gelatin capsules of suitable size with either 200 mg or 400 mg in each capsule.

**Example 4.**

Preparation of an antiviral composition in the form of compositions for intramuscular, intraperitoneal, or subcutaneous injections. The prodrug of formula 1.1 (500 mg) was mixed with chlorobutanol (300 mg), propylene glycol (2 ml), and injectable water. The resulting solution was filtered, placed into 5 ml ampoules, and sealed.

**Example 5.**

Evaluation of anti-HCV activity and cytotoxicity for compositions comprising the prodrug of formula 1.1 and Sovaldi®. The antiviral activity of tested compositions comprising the prodrug was evaluated using an Huh7 human hepatocellular carcinoma cell line stably transfected with an HCV replicon. A cell suspension in a complete culture medium (DMEM 1X, Cellgro; cat. # 10-013-CV) was transferred into 96-well plates (50 μl per well) with a final density of 7500 cells per well. The serial dilutions of tested prodrugs were prepared from a fresh 200-fold generic solution in DMSO with 11 concentration points and 3-fold dilution for each step starting from 20 nM in a complete medium and were used as 2-fold solutions in two replicates. Minimum 4 hours after planting the cells, 50 μl of serial prodrug dilutions was added to each well. The final prodrug concentration varied from 10 nM to 0.1 pM and that of DMSO, 0.5%. The plate with cells was incubated for 3 days at 37°C under humidified 5% $CO_2$. Upon completion of incubation, the medium was removed by turning the plate over and shaking it carefully. The cells were fixed for one minute with 100 μl of a 1:1 acetone:methanol solution, washed 3 times with a phosphate buffer (PBS), and blocked for 1 h at room temperature using a 10% fetal bovine serum (FBS) in PBS (150 μl/well). The cells were washed 3 times with PBS and incubated for 2 h at 37°C with anti-NS5B HCV antibodies (100 μl/well) using Affinity BioReagents (cat. # MA1-080) and diluting the generic solution (1 mg/ml) in a ratio of 1:4000 in 10% FBS-PBS. The cells were washed 3 times with PBS and developed by an OPD solution (100 μl/well) using for each plate one OPD tablet dissolved in a citrate/phosphate buffer (12 ml)), whereto 5 μl of 30% $H_2O_2$ was added, for 30 minutes in the dark at room temperature. The reaction was stopped by 2N $H_2SO_4$ (100 μl/well), and OD490 was measured using the multifunctional reader Victor$^3$ V 1420 (Perkin Elmer). The values of $EC_{50}$ for tested prodrugs were measured from an activity curve plotted using the GraphPad Prizm software. Notably, in comparable conditions Sovaldi® has against the genotype 1b (gT1b) HCV in a medium of 10% fetal bovine serum (10% FBS) $EC_{50}$ 45.0-170.0 nM and $EC_{90}$ 520.0 nM, whereas the novel prodrug of formula 1.1 has $EC_{50}$ 15.0-27.0 nM and $EC_{90}$ 128.0 nM (Table 2). Consequently, the new prodrug of formula 1.1 is more than three times more active than Sovaldi®.

**Table 2.**

| Inhibiting activities of prodrug 1.1 and Sovaldi® against gT1b NS5B HCV | | | |
|---|---|---|---|
| Prodrug | 10% FBS | | |
| | $EC_{50}$, nM | $EC_{90}$, nM | $CC_{50}$, μM |
| of formula 1.1 | 15.0-27.0 | 128.0 | >100 |
| *Sovaldi® | 45.0-170.0* | 520.0** | >100** |
| See *http//www.hcvdruginfo.ca/HCV_Sofosbuvir.pdf; ** M. J. Sofia et al. J. Med. Chem. 2010, 53, 7202-7218. | | | |

The cytotoxicity of tested compositions comprising the prodrug was evaluated concurrently in the same cell line Huh7 using an ATPLite kit (Perkin-Elmer, Boston, USA) in accordance with the manufacturer's instruction. The cell suspension in complete culture medium (DMEM 1X, Cellgro; cat. # 10-013-CV) was transferred into 96-well plates with black walls and transparent bottoms (50 μl/well) with a final density of 7500 cells per well. Eighteen hours after planting the cells, serial compound dilutions (50 μl/well) were added. The plate with cells was incubated for 4 days at 37°C in a humidified 5% $CO_2$ atmosphere. The cells were then twice washed with PBS (200 μl/well) and lysed by adding a lytic buffer (50 μl/well); all reagents were taken from the ATPLite kit. Following a 5-minute stirring on a shaker, a substrate was added (50 μl/well). After an additional 5-minute incubation, the plate was kept for 10 minutes in the dark, and luminescence in the wells was measured using the multifunctional reader Victor$^3$ V 1420 (Perkin Elmer). The values of $CC_{50}$ for tested compounds were measured from a cytotoxicity curve plotted using the GraphPad Prizm software. In particular, for the new prodrug of formula 1.1 and Sovaldi®, cytotoxicity values were found to be $CC_{50}$ > 100 μM (Table 2) and those of therapeutic window (therapeutic index TI = $EC_{50}/CC_{50}$), TI > 6 000.0.

**Example 6.**

Evaluation of kinetic solubility for compounds.

*Principle of the method.* The tested compound was dissolved in DMSO to reach a 10-mM concentration and then poured into an aqueous solvent (a phosphate buffer, water, or universal buffers of different pH values) to bring the concentration down to 200 μM. The resulting solution placed in a 96-well filter plate (Millipore's MultiScreen Solubility Filter Plate) was incubated for an hour at room temperature on a shaker, and the residue was filtered off *in vacuo.* The absorption spectrum of the compound was recorded on a spectrophotometer in a range of 240-400 nm with a 10-nm increment. For quantitative estimation of solubility, a calibration curve of standard solutions (0 - 200 μM) comprising 40% acetonitrile was used. The range of estimated concentrations was 3-200 μM. The test procedure was performed in duplicate.

*Preparation of calibration standards.* Calibration standards were prepared from 50-fold stock solutions in DMSO diluted in a buffer with 40% acetonitrile, which was added to ensure complete solubility of the tested compound in the calibration sample. Six standard samples with concentrations of 0, 3.125, 12.5, 50, 100, and 200 μM were prepared in the wells of a UV 96-well plate by adding 4 μl of corresponding 50x stock solutions in DMSO to 196 μl of a buffer comprising 40% acetonitrile. The concentration of DMSO in all points was constant and equal to 2% (v/v).

To plot calibration curves, the optical spectrum of the UV plate was recorded in a wavelength range of 250-400 nm with a 10-nm increment. Based on the spectral data for each compound, wavelengths were selected to meet the following criteria:

- For minimum compound concentration, OD > 0.1 (AU);
- For maximum compound concentration, OD < 2.0.

For each compound, a calibration curve was plotted with OD at selected wavelength as a function of concentration.

*Evaluation of kinetic solubility for compounds.* Solubility was evaluated in a MultiScreen Solubility (Millipore Corp.) filter plate as follows:

- Into each well of the MultiScreen Solubility filter plate, 196 μl of a buffer (without acetonitrile) and 4 μl of a 10-mM compound in DMSO or 4 μl of DMSO (for a blank matrix) were added. The plate was incubated for one hour on a shaker (400 rpm) at room temperature.
- The resulting solutions were filtered off through the filter plate by means of vacuum (10" Hg) into a polypropylene plate with a U-shaped bottom.
- From the U-bottom plate, the filtrate (120 μl/well) was transferred into a new UV plate, where to acetonitrile (80 μl/well) was added.
- The optical density of resulting solutions for a pre-selected wavelength was measured for each compound.

*Calculations.* The final compound concentration in the filtrate was computed as follows:

$$C_{filtrate} = (OD_{\lambda}Filtrate - OD_{\lambda}Blank)/Slope \times 1.67,$$

wherein

- $OD_\lambda$Filtrate is the optical density of filtrate for a selected wavelength;
- $OD_\lambda$Blank is OD of a blank matrix;
- Slope is the gradient of the calibration line;
- 1.67 is dilution factor for the filtrate diluted with acetonitrile.

The findings are presented in Table 1.

**Example 7.**

X-ray powder phase analysis of prodrug samples All diffraction patterns were recorded on a Bruker D8 Advance Vario diffractometer equipped with a copper-anode X-ray tube and a Ge(111) monochromator (CuKo^) and a LynxEye position-sensitive detector, in peek-a-boo settings. The shot range was 3-90° ***26*** for sample s5 and 5.7-90° ***26*** for the other samples, and the increment was 0.01° ***26***. The analysis was carried out using the Bruker Topas5 software ['Bruker TOPAS 5 User Manual. - Karlsruhe, Germany : Bruker AXS GmbH, 2015.].
Samples obtained by recrystallization of compound 1 from a mixture of ethyl acetate and methyl-*tert*-butyl ether (1:1), ethanol, ethyl acetate and a mixture of acetic acid with water had polycrystalline forms. X-ray powder phase analyses of these samples revealed a similarity of their qualitative phase structures and an insignificant difference in their phase relations. The samples had an orthorhombic phase with the following unit cell parameters: ***a*** = 28.1056(8) A, ***b*** = 16.8998(4) A, and ***c*** = 5.25380(12) A. Systematic extinctions allow one to assume a spatial group of $P2_1$. A unit cell volume of 1266.98(9) $A^3$ corresponds to the claimed composition and Z' = 1. The evaluation of phase relations based on comparisons of integral peak intensities suggests that the content of monoclinic phase varies from 30 to 50%.
The sample obtained by recrystallization of compound 1 from a mixture of dimethyl sulfoxide with water is a white substance of crystalline form. According to X-ray powder analysis data, the sample of said form is single-phase and consists of an orthorhombic phase with the following unit cell parameters: ***a*** = 28.1056(8) A, ***b*** = 16.8998(4) A, ***c*** = 5.25380(12) A. Systematic extinction analysis allows one to assume a spatial group of $P2_12_12_1$. A unit cell volume of 2495.45(11) $A^3$ corresponds to the claimed composition and Z' = 1.

**Example 8.**

Evaluation of stability in biological matrix for compositions comprising the prodrug of formula 1.1. Initial compositions comprising the prodrug of formula 1.1 (the tested compounds) were prepared with a concentration of 10 mM in DMSO. From said compositions, 100-fold working solutions were prepared with a concentration of 100 μM in a mixture of acetonitrile : water with a volumetric ratio of 1:1.

a) Stability in S9 fraction. The reaction mixture was prepared in a 0.1 M potassium phosphate buffer (pH 7.4 BD Gentest) in a total final volume of 250 μl and contained a 1 mM NADPH-tetrasodium salt (AppliChem), 7 mM glucose-6-phosphate sodium salt (Sigma), 1.5 U/ml glucose-6-phosphate dehydrogenase (Sigma), 3.3 mM $MgCl_2$ (Sigma), 5 mM uridine-5-diphosphate-glucuronic acid trisodium salt (UDPGluA, Sigma), and 1 μM tested compound (these are final concentrations). The metabolic reaction was initiated by adding a suspension of human liver S9 fraction (BD Gentest), and the final concentration of protein was 1 mg/ml. The reaction mixture was incubated at 37°C on a Vortemp56 shaker with stirring at 400 rpm. After certain intervals (0, 0.25, 0.5, 1, 2, 4, 6, 8, 24 h), 30-μl samples were taken; the reaction was stopped by adding cold acetonitrile (180 μl) comprising an internal standard to the sample taken. Proteins were deposited on ice for 15 min. The samples were then centrifuged for 10 min at 3000 rpm, and the supernatant (150 μl) was sampled for analysis. The incubation was performed in two replicates, with each sample measured twice.

b) Stability in artificial gastric and intestinal juices. The tested composition comprising the prodrug of formula 1.1 in a final concentration of 1 μM was incubated in artificial gastric juice (0.2% NaCl in 0.7% v/v HCl) and artificial intestinal juice (0.05M $KH_2PO_4$, pH 6.75). The incubation was performed in a Vortemp56 shaking incubator at 37°C with stirring at a rate of 300 rev/min. After certain intervals (0, 0.25, 0.5, 1, 2, 4, 6, 8, 24 h), a 30 μl sample was taken; the reaction was stopped by adding cold acetonitrile (180 μl) comprising an internal standard to the sample taken. The samples were then centrifuged, and the supernatant (150 μl) was sampled for analysis for 10 min at 3000 rev/min. The incubation was performed in two replicates, with each sample measured twice.

c) Stability in blood plasma. The tested compound in a final concentration of 1 μM was incubated in pooled human blood plasma (Innovative Research). The incubation was performed in a Vortemp56 shaking incubator at 37°C with stirring at a rate of 300 rev/min. After certain intervals (0, 0.25, 0.5, 1, 2, 4, 6, 8, 24 h), a 30 μl sample was taken; the reaction was stopped by adding cold acetonitrile (180 μl) comprising an internal standard to the sample taken.

The samples were then centrifuged, and the supernatant (150 μl) was sampled for analysis for 10 min at 3000 rev/min. The incubation was performed in two replicates, with each sample measured twice.

*Sample analysis.* Samples were analyzed using an HPLC-MS/MS technique developed for each tested prodrug, wherein the chromatographic system 1290 Infinity II (Agilent Technologies) was combined with the tandem mass spectrometer QTRAP5500 (AB Sciex). When developing conditions for mass-spectrometric detection, the solutions of tested compounds in a mixture of acetonitrile-water (1:1) with a concentration of 100 ng/ml were injected directly into the mass spectrometer using a syringe pump with electrospray ionization carried out in a positive ion mode. Scanning in a total ion current mode (MS1) allowed us to identify a molecular ion for each compound, and basic product ions were recorded in MS2 mode. Then, to attain maximum sensitivity, the MS/MS technique was optimized in MRM mode. In quantitative chromatogram processing, the most intensive MRM transition was used for the analyte and the internal standard. Separation was carried out by means of linear gradient elution chromatography on a YMC Triart C18 column (50 x 2 mm, 1.9 μm) in a mobile phase consisting of 0.1% formic acid in water and 0.1% formic acid in acetonitrile. Tolbutamide (Fluka) was used as the internal standard.

*Computations.* Half-life ($T_{1/2}$) was found from the kinetics of tested prodrug elimination in an antiviral composition during incubation in a biological matrix. The computations were based on the values of chromatographic peak areas for compounds in test samples normalized to the internal standard signal. From linear dependence of log normalized areas of chromatographic peaks on time, the constant of elimination rate was calculated (k is linear section slope). Then, half-life was found: $T_{1/2}$ = 0.693 / k. It was discovered, in particular (see Table 3), that the prodrugs of formulas 1.1, 1.2, and Sovaldi® in the antiviral composition have comparable stabilities in human gastric juice ($T_{1/2}^{SGF}$ = 12.7-15 h), in human intestinal juice ($T_{1/2}^{SIF}$ > 24 h), and in human plasma ($T_{1/2}^{HPL}$ > 24 h). At the same time, the prodrug of formula 1.1 more actively metabolizes in human liver microsomal S9 fraction and has half-life $T_{1/2}^{HS9}$ = 0.05 h, while its prototype Sovaldi® has $T_{1/2}^{HS9}$ = 0.54 h (Table 3), which means that the prodrug of formula 1.1 metabolizes in human liver microsomal S9 fraction 11 times faster than Sovaldi®.

**Table 3.**

| Stability and activity of antiviral compositions comprising the novel prodrug of formula 1.1 and Sovaldi® | | | | |
|---|---|---|---|---|
| ID | $T_{1/2}$ (h) | | | |
| | SGF | SIF | Human plasma | Human S9 |
| 1.1 | 12.7 | >24 | >24 | 0.05 |
| Sovaldi® | 22.0* | >24* | >24* | 0.57* |
| *See M. J. Sofia et al. J. Med. Chem. 2010, 53, 7202-7218. | | | | |

**Example 9.**

Pharmacokinetic (PK) study of compositions comprising the prodrug of formula 1.1 and Sovaldi® in rat liver.

*Preparation of compositions comprising the prodrug of formula 1.1 and Sovaldi® for administration to rats.* The tested composition was administered at a dose of 50 mg/kg. To this end, compositions were prepared with a concentration of prodrug 1.1 or Sovaldi® of 5.0 mg/ml in a 0.5% solution of hydroxypropyl methylcellulose (HPMC), to which 5% ethanol was added, as follows: to a weighed portion of the prodrug of formula 1.1 or Sovaldi®, an appropriate amount of HPMC was added, and the mixture was triturated dry in a mortar, whereafter a proper quantity of 5% ethanol in distilled water was gradually added portionwise, and the mixture was carefully stirred to obtain a suspension suitable for intragastric administration.

*Administration of compositions comprising the prodrug of formula 1.1 and Sovaldi® to animals. Preparation of blood plasma and liver samples.* The study was carried out on Sprague Dawley rats. The rats were divided into groups of 6 based on selected time points (1, 2, 4, 6, 8, 10, 12, 16, and 24 h). The rats were weighed, and the volume of compositions comprising the prodrug of formula 1.1 or Sovaldi® was calculated for each animal at a rate of 10 ml/kg. Compositions comprising the prodrug of formula 1.1 or Sovaldi® were administered intragastrically through a feeding tube. In the intervals between administrations to the animals of a certain group, samples of liver and blood were taken. After an appropriate period of time following the administration, the rat was euthanized by $CO_2$ inhalation. Immediately after the euthanasia, the animal was quickly opened up, and its upper lobe of the liver was cut and instantaneously placed in liquid nitrogen. The frozen liver fragment was then transferred into a labeled test tube cooled with liquid nitrogen. The samples were kept in liquid nitrogen till the end of the experiment and then put into an ultra-cold freezer at -80°C.

*Sample preparation.* A liver sample weighing about 1 g was triturated in a mortar while being cooled with liquid

nitrogen. The resulting powder was poured over with triple-volume of methanol with 70% EDTA methanol and was twice homogenized for 45 s (with a 10-second interval) at a rate of 6.3 m/s using the Omni Bead Ruptor 24 homogenizer. To 360 μl of thus obtained homogenate, 40 μl of ten-fold standard solution comprising PSI-7409 and H027-4261 (or methanol, in case of experimental samples) and 100 μl of internal standard solution (5-bromouridine triphosphate) with a concentration of 25 ng/ml were added. After stirring and centrifuging, 400 μl of supernatant was diluted with 400 μl of a 1% formic acid solution in a mixture of methanol - water (1:1). Then, solid-phase extraction was performed using Waters Oasis WAX cartridges. The resulting product was eluted with 800 μl of a 5% solution of ammonia in methanol, and the eluate was evaporated and redissolved in 200 μl of methanol.

*HPLC-MS/MS analytical conditions.* Samples were analyzed using an HPLC-MS/MS technique, wherein the HPLC system Agilent 1290 Infinity II was combined with the AB Sciex QTrap 5500 mass spectrometer. Separation was carried out on a Thermo Hypercarb column (50 x 3 mm, 5 μm). A 25-mM solution of ammonium acetate with 0.5% ammonium was used as mobile phase A (MPA); a 25-mM solution of ammonium acetate in a mixture of water-isopropanol-acetonitrile (1:1:3) with 0.5% ammonium was used as mobile phase B (MPB). Separation was performed in gradient mode: 0-0.3 min - 5% MPB; 3-3.4 min - 50% MPB; 3.6-4.5 min - 5% MPB. PSI-7409 and H027-4261 were recorded in MRM mode with ion transitions of 499/159 and 410/150, respectively.

*Pharmacokinetic analysis.* Pharmacokinetic analysis of "liver concentration versus time" data was performed by a non-compartmental technique using Phoenix™ WinNonlin® 6.3 (Pharsight Corp.) and GraphPad Prizm software. The following pharmacokinetic parameters were computed: maximum concentration in liver ($C_{max}$) and time of achievement thereof ($T_{max}$), half-life ($T_{1/2}$), and area under the PK curve ($AUC_{0-t}$, $AUC_{0-inf}$). The findings are given in Table 4. As can be seen from Table 4, the concentration and $AUC_{24h}$ of triphosphate PSI-7409 resulting from the metabolism of the prodrug of formula 1.1 in the rat liver are $C_{max}$ =3224.0 ng/g and $AUC_{24\ h}$ = 30487.0 ngh/g, respectively, while Sovaldi® exhibiting similar metabolism has $C_{max}$ =1934.0 ng/g and $AUC_{24\ h}$ = 16,796.0 ng.h/g (Table 4). This suggests that the novel prodrug is almost twice more effective in its liver metabolism into target triphosphate PSI-7409 (drug).

**Table 4.**

| Pharmacokinetic (PK) parameters of triphosphate PSI-7409 in the rat liver following peroral administration of the prodrug of formula 1.1 and Sovaldi® in a dose of 50 mg/kg | | | |
|---|---|---|---|
| PK parameters | Software used for processing PK results | | According to M. J. Sofia et al. J. Med. Chem. 2010, 53, 7202-7218. |
| | Phoenix™ WinNonlin® 6.3 | GraphPad Prizm | |
| | Prodrug of formula 1.1 | | Sovaldi® |
| $T_{1/2}$, h | 7.2 | 5.5 | |
| $T_{max}$, h | 8.0 | 4.0 | 4.0 |
| $C_{max}$, ng/g | 3224.0 | 3102.0 | 1934 |
| $C_{24\ h}$, ng/g | 320.0 | | |
| $AUC_{24\ h}$, ngh/g | 30487.0 | 30444.0 | 16796.0 |
| $AUC_{0-inf}$, ngh/g | 33823.0 | | 18080.0 |

## Industrial applicability

The invention could be used in medicine and veterinary.

## Claims

1. An antiviral pharmaceutical composition for a combination treatment of hepatitis C virus in a subject in need thereof, said composition comprising an effective amount of a compound selected from cyclobutyl (*S*)-2-{[(2*R*,3*R*,4*R*,5*R*)-5-(2,4-dioxo-3,4-dihydro-2H-pyrimidin-1-yl)-4-fluoro-3-hydroxy-4-methyl-tetrahydrofuran-2-ylmethoxy]-phenoxy-phosphorylamino}-propanoate of general formula **1,** and/or cyclobutyl (*S*)-2-{(*S*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydro-furan-2-ylmethoxy]-phenoxy-phosphorylamino} -propanoate of formula **1.1,** and/or cyclobutyl (*S*)-2-{(*R*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydro-furan-2-ylmethoxy]-phenoxy-phosphorylamino}-propanoate of formula **1.2,** or an isotopically enriched, crystalline or polycrystalline form thereof

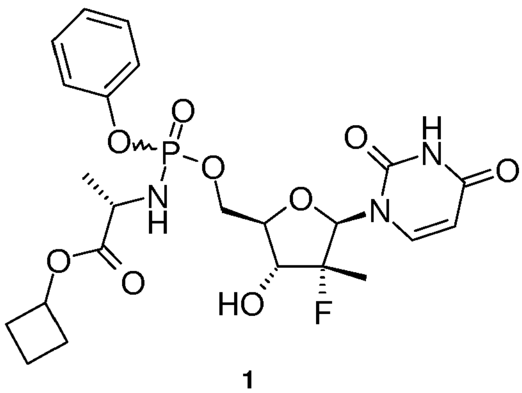

**1**

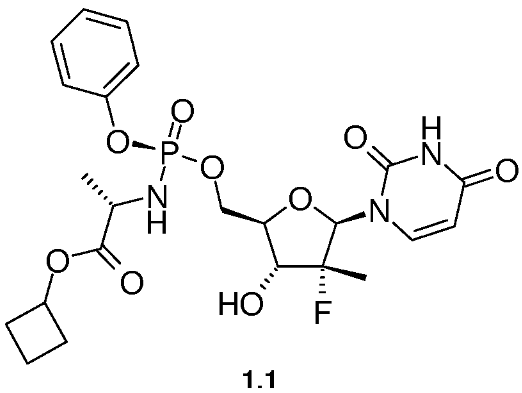

**1.1**

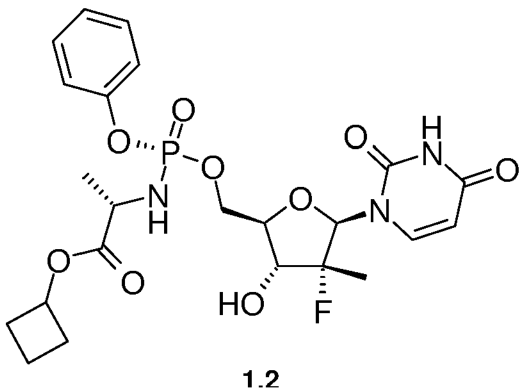

**1.2**

and a pharmaceutically acceptable carrier.

**2.** The antiviral pharmaceutical composition according to Claim 1, additionally comprising a therapeutically effective amount of an additional active agent selected from an HCV NS5A inhibitor, an HCV NS3 inhibitor, an HCV NS3/4A inhibitor an HBV DNA polymerase inhibitor, an HIV 1 reverse transcriptase inhibitor.

**3.** The antiviral pharmaceutical composition according to Claim 1, additionally comprising a therapeutically effective amount of an HBV DNA polymerase inhibitor and an HIV 1 reverse transcriptase inhibitor.

**4.** The antiviral pharmaceutical composition according to Claims 1 or 2 comprising a NS5A inhibitor selected from Daclatasvir (BMS-790052), Hepavivir (AV-4025), AV-4067, AV-4084, AV-4056, AV-4058, Ombitasvir (ABT-267), Elbasvir (MK-8742) or Velpatasvir (VEL, GS-5816).

**5.** The antiviral pharmaceutical composition according to Claims 1 or 2 comprising Narlaprevir (SCH 900518) as an NS3 inhibitor.

**6.** The antiviral pharmaceutical composition according to Claims 1 or 2 comprising Simeprevir (*Olysio*) as an NS3/4A inhibitor.

**7.** A combination of active agents, comprising a compound selected from cyclobutyl (*S*)-2-{[(2*R*,3*R*,4*R*,5*R*)-5-(2,4-dioxo-3,4-dihydro-2H-pyrimidin-1-yl)-4-fluoro-3-hydroxy-4-methyl-tetrahydrofuran-2-ylmethoxy]-phenoxy-phosphorylamino}-propanoate of general formula **1,** and/or cyclobutyl (*S*)-2-{(*S*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydro-furan-2-ylmethoxy]-phenoxy-phosphorylamino} -propanoate of formula **1.1,** and/or cyclobutyl (*S*)-2-{(*R*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydro-furan-2-ylmethoxy]-phenoxy-phosphorylamino}-propanoate of formula **1.2,** or an isotopically enriched, crystalline or polycrystalline form thereof, and one or more anticancer agents and/or other antiviral agents for use in combination therapy of hepatitis C.

**8.** The combination of active agents for use according to Claim 7, wherein the combination therapy of hepatitis C in a subject in need thereof includes successive or simultaneous administration of a therapeutically effective amount of

the compound of general formula **1,** and/or formula **1.1,** and/or formula 1.2, and or an isotopically enriched, crystalline or polycrystalline form thereof, and of the one or more anticancer agents and/or other antiviral agents.

**9.** The combination of active agents for use according to Claim 7, wherein the other antiviral agent is selected from Daclatasvir (BMS-790052), Hepavivir (AV-4025), AV-4067, AV-4084, AV-4056, AV-4058, Ombitasvir (ABT-267), Elbasvir (MK-8742) or Velpatasvir (VEL, GS-5816).

**10.** The combination of active agents for use according to Claim 7, wherein the other antiviral agent is selected from a NS3 inhibitor Narlaprevir (SCH 900518), a NS3/4A inhibitor Simeprevir (*Olysio*), an inhibitor polymerase DNA HBV, and an HIV 1 reverse transcriptase inhibitor.

**11.** The combination of active agents for use according to Claim 7, comprising an HBV DNA polymerase inhibitor and an HIV 1 reverse transcriptase inhibitor as said other antiviral agent.

**12.** The combination of active agents for use according to Claim 7 comprising one or more anticancer agents.

**Patentansprüche**

**1.** Antivirale pharmazeutische Zusammensetzung zur Kombinationsbehandlung des Hepatitis C-Virus bei einem Subjekt, das diese benötigt, wobei die Zusammensetzung eine wirksame Menge einer Verbindung umfasst, ausgewählt aus Cyclobutyl-(*S*)-2-{[(2*R*,3*R*,4*R*,5*R*)-5-(2,4-dioxo-3,4-dihydro-2H-pyrimidin-1-yl)-4-fluor-3-hydroxy-4-methyl-tetrahydrofuran-2-ylmethoxy]-phenoxy-phosphorylamino}-propanoat der allgemeinen Formel **1** und/oder Cyclobutyl-*(S)*-2-{(*S*)-[(2*R*,3*R*,4*R*,5*R*)-5-(3,4-dihydro-2,4-dioxo-2*H*-pyrimidin-1-yl)-3-hydroxy-4-methyl-4-fluor-tetrahydrofuran-2-ylmethoxy]-phenoxy-phosphorylamino}-propanoat der Formel **1.1** und/oder Cyclobutyl-(S)-2-{(R)-[(2*R*,3*R*,4*R*,5*R*)-5-(3,4-dihydro-2,4-dioxo-2*H*-pyrimidin-1-yl)-3-hydroxy-4-methyl-4-fluor-tetrahydro-furan-2-ylmethoxy]-phenoxy-phosphorylamino}-propanoat der Formel **1.2** oder einer isotopisch angereicherten, kristallinen oder polykristallinen Form davon

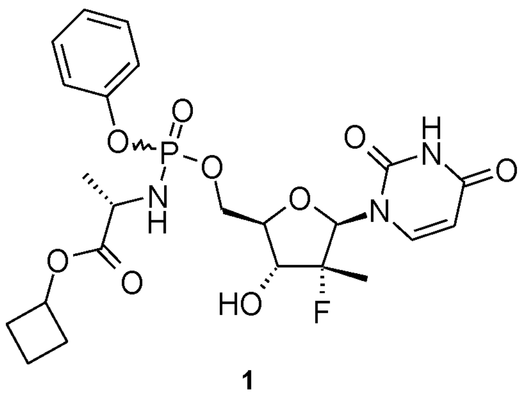

**1**

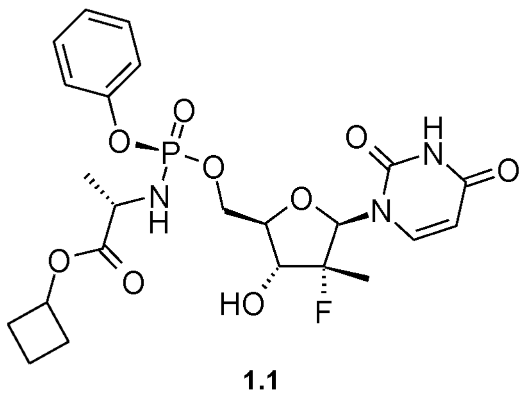

**1.1**

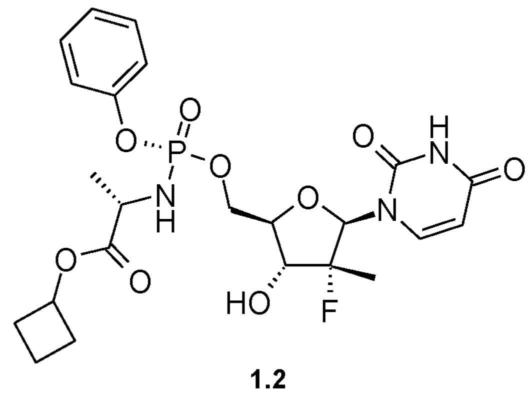

**1.2**

und einen pharmazeutisch akzeptablen Träger.

2. Antivirale pharmazeutische Zusammensetzung nach Anspruch 1, zusätzlich umfassend eine therapeutisch wirksame Menge eines zusätzlichen Wirkstoffes ausgewählt aus einem HCV NS5A-Inhibitor, einem HCV NS3-Inhibitor, einem HCV NS3/4A-Inhibitor, einem HBV DNA Polymerase-Inhibitor, einem HIV 1 Reverse Transkriptase-Inhibitor.

3. Antivirale pharmazeutische Zusammensetzung nach Anspruch 1, zusätzlich umfassend eine therapeutisch wirksame Menge eines HBV DNA Polymerase-Inhibitors und eines HIV 1 Reverse Transkriptase-Inhibitors.

4. Antivirale pharmazeutische Zusammensetzung nach Anspruch 1 oder 2, umfassend einen NS5A-Inhibitor, ausgewählt aus Daclatasvir (BMS-790052), Hepavivir (AV-4025), AV-4067, AV-4084, AV-4056, AV-4058, Ombitasvir (ABT-267), Elbasvir (MK-8742) oder Velpatasvir (VEL, GS-5816).

5. Antivirale pharmazeutische Zusammensetzung nach Anspruch 1 oder 2, umfassend Narlaprevir (SCH 900518) als NS3-Inhibitor.

6. Antivirale pharmazeutische Zusammensetzung nach Anspruch 1 oder 2, umfassend Simeprevir (*Olysio*) als NS3/4A-Inhibitor.

7. Wirkstoffkombination umfassend eine Verbindung ausgewählt aus Cyclobutyl-*(S)*-2-{[(2*R*,3*R*,4*R*,5*R*)-5-(2,4-dioxo-3,4-dihydro-2H-pyrimidin-1-yl)-4-fluor-3-hydroxy-4-methyl-tetrahydrofuran-2-ylmethoxy]-phenoxy-phosphorylamino}-propanoat der allgemeinen Formel **1** und/oder Cyclobutyl-*(S)*-2-{(*S*)-[(2*R*,3*R*,4*R*,5*R*)-5-(3,4-dihydro-2,4-dioxo-2*H*-pyrimidin-1-yl)-3-hydroxy-4-methyl-4-fluor-tetrahydro-furan-2-ylmethoxy]-phenoxy-phosphorylamino}-propanoat der Formel **1.1** und/oder Cyclobutyl-(*S*)-2-{(*R*)-[(2*R*,3*R*,4*R*,5*R*)-5-(3,4-dihydro-2,4-dioxo-2*H*-pyrimidin-1-yl)-3-hydroxy-4-methyl-4-fluor-tetrahydro-furan-2-ylmethoxy]-phenoxy-phosphorylamino}-propanoat der Formel **1.2** oder einer isotopisch angereicherten, kristallinen oder polykristallinen Form davon und einen oder mehrere Anti-Krebs-Wirkstoffe und/oder einen oder mehrere andere antivirale Wirkstoffe zur Verwendung in der Kombinationstherapie von Hepatitis C.

8. Wirkstoffkombination zur Verwendung nach Anspruch 7, wobei die Kombinationstherapie des Hepatitis C-Virus bei einem Subjekt, das diese benötigt, eine aufeinanderfolgende oder gleichzeitige Verabreichung einer therapeutisch wirksamen Menge der Verbindung der allgemeinen Formel **1.1** und/oder der Formel **1.1** und/oder der Formel 1.2 und oder einer isotopisch angereicherten, kristallinen oder polykristallinen Form davon und des einen oder der mehreren Anti-Krebs-Wirkstoffe(s) und/oder des einen oder der mehreren anderen antiviralen Wirkstoffe(s) umfasst.

9. irkstoffkombination zur Verwendung nach Anspruch 7, wobei der andere antivirale Wirkstoff ausgewählt ist aus Daclatasvir (BMS-790052), Hepavivir (AV-4025), AV-4067, AV-4084, AV-4056, AV-4058, Ombitasvir (ABT-267), Elbasvir (MK-8742) oder Velpatasvir (VEL, GS-5816).

10. Wirkstoffkombination zur Verwendung nach Anspruch 7, wobei der andere antivirale Wirkstoff ausgewählt ist aus einem NS3-Inhibitor Narlaprevir (SCH 900518), einem NS3/4A-Inhibitor Simeprevir (*Olysio*), einem HBV DNA Polymerase-Inhibitor und einem HIV 1 Reverse Transkriptase-Inhibitor.

11. Wirkstoffkombination zur Verwendung nach Anspruch 7, umfassend einen HBV DNA Polymerase-Inhibitor und einen HIV 1 Reverse Transkriptase-Inhibitor als den anderen antiviralen Wirkstoff.

12. Wirkstoffkombination zur Verwendung nach Anspruch 7, umfassend einen oder mehrere Anti-Krebs-Wirkstoffe.

## Revendications

1. Composition pharmaceutique antivirale destinée à un traitement combinatoire du virus de l'hépatite C chez un sujet qui en manifeste le besoin, ladite composition comprenant une quantité efficace d'un composé qui est choisi parmi le (*S*)-2-{[(2*R*,3*R*,4*R*,5*R*)-5-(2,4-dioxo-3,4-dihydro-2*H*-pyrimidin-1-yl)-4-fluoro-3-hydroxy-4-méthyl-tétrahydrofuran-2-ylméthoxy]-phénoxy-phosphorylamino}-propanoate de cyclobutyle répondant à la formule générale **1,** et/ou le (*S*)-2-{(*S*)-[(2*R*,3*R*,4*R*,5*R*)-5-(3,4-dihydro-2,4-dioxo-2*H*-pyrimidin-1-yl)-3-hydroxy-4-méthyl-4-fluoro-tétrahydro-furan-2-ylméthoxy]-phénoxy-phosphorylamino}-propanoate de cyclobutyle répondant à la formule **1.1,** et/ou le (*S*)-2-{(R)-[(2*R*,3*R*,4*R*,5*R*)-5-(3,4-dihydro-2,4-dioxo-2*H*-pyrimidin-1-yl)-3-hydroxy-4-méthyl-4-fluoro-tétrahydro-furan-2-ylméthoxy]-phénoxy-phosphorylamino}-propanoate de cyclobutyle répondant à la formule **1.2,** ou une de leurs formes cristallines ou polycristallines enrichies du point de vue isotopique

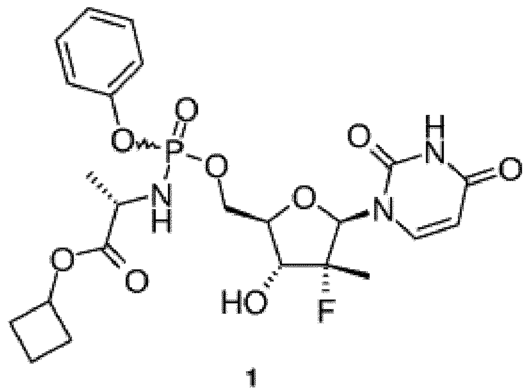

1

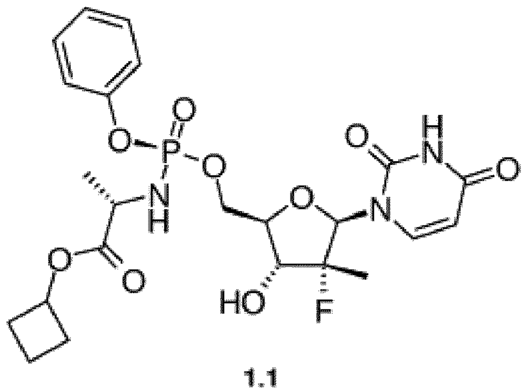

1.1

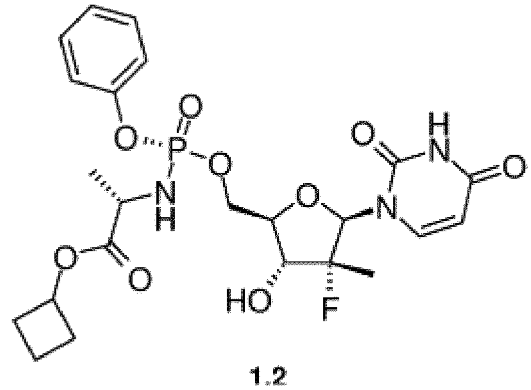

1.2

et un support pharmaceutiquement acceptable.

**2.** Composition pharmaceutique antivirale selon la revendication 1, qui comprend en outre une quantité efficace du point de vue thérapeutique d'un agent actif supplémentaire qui est choisi parmi un inhibiteur de la protéine NS5A du VHC, un inhibiteur de la protéine NS3 du VHC, un inhibiteur de la protéine NS3/4A du VHC, un inhibiteur de l'ADN polymérase du VHB, un inhibiteur de la transcriptase inverse du VIH 1.

**3.** Composition pharmaceutique antivirale selon la revendication 1, qui comprend en outre une quantité efficace du point de vue thérapeutique d'un inhibiteur de l'ADN polymérase du VHB et un inhibiteur de la transcriptase inverse du VIH 1.

**4.** Composition pharmaceutique antivirale selon la revendication 1 ou 2, qui comprend un inhibiteur de la protéine NS5A, qui est choisi parmi le daclatasvir (BMS-790052), l'hépavivir (AV-4025), AV-4067, AV-4084, AV-4056, AV-4058, l'ombitasvir (ABT-267), l'elbasvir (MK-8742) ou le velpatasvir (VEL, GS-5816).

**5.** Composition pharmaceutique antivirale selon la revendication 1 ou 2, qui comprend du narlaprevir (SCH 900518) à titre d'inhibiteur de la protéine NS3.

**6.** Composition pharmaceutique antivirale selon la revendication 1 ou 2, qui comprend du simeprevir (*Olysio*) à titre d'inhibiteur de la protéine NS3/4A.

**7.** Combinaison d'agents actifs, qui comprend un composé choisi parmi le (*S*)-2-{[(2*R*,3*R*,4*R*,5*R*)-5-(2,4-dioxo-3,4-dihydro-2*H*-pyrimidin-1-yl)-4-fluoro-3-hydroxy-4-méthyl-tétrahydrofuran-2-ylméthoxy]-phénoxy-phosphorylamino}-propanoate de cyclobutyle répondant à la formule générale **1,** et/ou le (*S*)-2-{(*S*)-[(2*R*,3*R*,4*R*,5*R*)-5-(3,4-dihydro-2,4-dioxo-2*H*-pyrimidin-1-yl)-3-hydroxy-4-méthyl-4-fluoro-tétrahydro-furan-2-ylméthoxy]-phénoxy-phosphorylamino}-propanoate de cyclobutyle répondant à la formule **1.1,** et/ou le (*S*)-2-{(*R*)-[(2*R*,3*R*,4*R*,5*R*)-5-(3,4-dihydro-2,4-dioxo-2*H*-pyrimidin-1-yl)-3-hydroxy-4-méthyl-4-fluoro-tétrahydro-furan-2-ylméthoxy]-phénoxy-phosphorylamino}-propanoate de cyclobutyle répondant à la formule **1.2,** ou une de leurs formes cristallines ou polycristallines enrichies du point de vue isotopique, et un ou plusieurs agents anticancéreux et/ou un ou plusieurs autres agents antiviraux pour leur utilisation dans une thérapie combinatoire de l'hépatite C.

8. Combinaison d'agents actifs pour son utilisation selon la revendication 7, dans laquelle la thérapie combinatoire de l'hépatite C chez un sujet qui en manifeste le besoin englobe une administration successive ou simultanée d'une quantité efficace du point de vue thérapeutique du composé répondant à la formule générale **1,** et/ou à la formule **1.1,** et/ou à la formule **1.2,** et/ou une de leurs formes cristallines ou polycristallines enrichies du point de vue isotopique, et desdits un ou plusieurs agents anticancéreux et/ou desdits un ou plusieurs autres agents antiviraux.

9. Combinaison d'agents actifs pour son utilisation selon la revendication 7, dans laquelle l'autre agent antiviral est choisi parmi le daclatasvir (BMS-790052), l'hépavivir (AV-4025), AV-4067, AV-4084, AV-4056, AV-4058, l'ombitasvir (ABT-267), l'elbasvir (MK-8742) ou le velpatasvir (VEL, GS-5816).

10. Combinaison d'agents actifs pour son utilisation selon la revendication 7, dans laquelle l'autre agent antiviral est choisi parmi le narlaprevir (SCH 900518) à titre d'inhibiteur de la protéine NS3, le simeprevir (*Olysio*) à titre d'inhibiteur de la protéine NS3/4A, un inhibiteur de l'ADN polymérase du VHB et un inhibiteur de la transcriptase inverse du VIH 1.

11. Combinaison d'agents actifs pour son utilisation selon la revendication 7, qui comprend un inhibiteur de l'ADN polymérase du VHB et un inhibiteur de la transcriptase inverse du VIH 1 à titre desdits autres agents antiviraux.

12. Combinaison d'agents actifs pour son utilisation selon la revendication 7, qui comprend un ou plusieurs agents anticancéreux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7964580 B
- US 8334270 B
- WO 2008021927 A
- WO 2008021928 A
- WO 2008021936 A
- WO 2010132601 A
- WO 2010144646 A
- WO 2012040923 A
- WO 2012041014 A
- WO 2015110048 A
- WO 2012074437 A
- US 9428491 B
- US 14845333 B
- WO 2011075615 A
- RU 2478104
- US 20140296136 A
- US 8987195 B
- US 7973040 B
- US 2012214783 A
- EP 1497282 A
- EP 2364984 A
- WO 2015023958 A
- WO 2012097012 A
- WO 2014106019 A
- WO 2014033176 A
- WO 2014033170 A
- WO 2014033167 A
- WO 2013006394 A
- US 20090163545 A


### Non-patent literature cited in the description


- Hepatitis C. WHO, July 2016
- **MESSINA, J. P.** Global Distribution and Prevalence of Hepatitis C Virus Genotypes. *Hepatology,* 2015, vol. 61 (1), 77-87
- **SOFIA, M. J. et al.** Discovery of a β-D-20-Deoxy-20-rfluoro-2 0-β-C-methyluridine Nucleotide Prodrug (PSI-7977) for the Treatment of Hepatitis C Virus. *J. Med. Chem.,* 2010, vol. 53, 7202-7218
- **BELEMA, M. et al.** Discovery and Development of Hepatitis C Virus NS5A Replication Complex Inhibitors. *J. Med. Chem.,* 2014, vol. 57, 1643-1672
- **LINK, J. O. et al.** Discovery of ledipasvir (GS-5885): a potent, once-daily oral NS5A inhibitor for the treatment of hepatitis C virus infection. *J. Med. Chem.,* 2014, vol. 57, 2033-2046
- **GARDELLI, C. et al.** Phosphoramidate Prodrugs of 20-C-Methylcytidine for Therapy of Hepatitis C Virus Infection. *J. Med. Chem.,* 2014, vol. 57, 2047-2057
- **COBURN, C. A. et al.** *ChemMedChem.,* 2013, vol. 8, 1930-1940
- **IVACHTCHENKO, A. V. et al.** Discovery of Novel Highly Potent Hepatitis C Virus NS5A Inhibitor (AV4025). *J. Med. Chem.,* 2014, vol. 57, 7716-7730
- **BILELLO J. P. et al.** In vitro activity and resistance profile of samatasvir, a novel NS5A replication inhibitor of hepatitis C virus. *Antimicrobobial Agents Chemother.,* 2014, vol. 58 (8), 4431-4442
- **NAKAMOTO S. et al.** Hepatitis C virus NS5A inhibitors and drug resistance mutations. *World J. Gastroenterol.,* 2014, vol. 20 (11), 2902-2912
- **ARASAPPAN A. et al.** Discovery of Narlaprevir (SCH 900518): A Potent, Second Generation HCV NS3 Serine Protease Inhibitor. *ACS Med. Chem. Lett.,* 2010, vol. 1 (2), 64-69
- **E. MURAKAMI et al.** Mechanism of activation of PSI-7851 and its diastereoisomer PSI-7977. *J. Biol. Chem.,* 2010, vol. 285 (45), 34337-34347
- **M. J. SOFIA et al.** Discovery of a β-D-20-Deoxy-20-r-fluoro-20-β-C-methyluridine Nucleotide Prodrug (PSI-7977) for the Treatment of Hepatitis C Virus. *J. Med. Chem.,* 2010, vol. 53, 7202-7218
- **I.M. JACOBSON et al.** Sofosbuvir for hepatitis C genotype 2 or 3 in patients without treatment options. *Engl. J. Med.,* 2013, vol. 368, 1867-1877
- **E. LEWIRZ et al.** Sofosbuvir for previously untreated chronic hepatitis C infection. *Engl. J. Med.,* 2013, vol. 368, 1878-1887
- **C. WANG et al.** Hepatitis C virus RNA elimination and development of resistance in replicon cells treated with BMS-790052. *Antimicrob. Agents Chemother.,* 2012, vol. 56, 1350-1358, https://en.wikipedia.org/wiki/Daclatasvir
- **A.V. IVACHTCHENKO et al.** Discovery of Novel Highly Potent Hepatitis C Virus NS5A Inhibitor (AV4025). *J. Med. Chem.,* 2014, vol. 57, 7716-7730

- **M. J. SOFIA et al.** *J. Med. Chem.,* 2010, vol. 53, 7202-7218
- Bruker TOPAS 5 User Manual. Bruker AXS GmbH, 2015